# EUROPEAN PATENT SPECIFICATION

(11) **EP 1 633 765 B1**
(45) Date of publication and mention of the grant of the patent: **14.11.2007**
(21) Application number: 04732099.9
(22) Date of filing: 11.05.2004
(51) Int. Cl.: C07H 17/08, A61K 31/7048

(54) **NOVEL 14 AND 15 MEMBERED-RING COMPOUNDS**
NEUE 14- UND 15-GLIEDRIGE RINGVERBINDUNGEN
NOUVEAUX COMPOSES A CYCLES A 14 ET 15 ELEMENTS

(30) Priority: 13.05.2003 GB 0310992
(43) Date of publication of application: 15.03.2006
(73) Proprietor: GLAXO GROUP LIMITED, Greenford, Middlesex UB6 0NN (GB); GlaxoSmithKline istrazivacki centar Zagreb d.o.o., 10000 Zagreb (HR)
(72) Inventor: ALIHODZIC, Sulejman, Pliva-Istrazivacki Inst. doo, 10000 Zagreb (HR); BERDIK, Andrea, Pliva-Istrazivacki Institut doo, 10000 Zagreb (HR); JARVEST, Richard, Lewis, GlaxoSmithKline, Stevenage, Hertfordshire SG1 2NY (GB); LAZAREVSKI, Gorjana, Pliva-Istrazivacki Inst. doo, 10000 Zagreb (HR)
(74) Representative: Hambleton, Bernadette Angelina
(86) International application number: PCT/EP2004/005086
(87) International publication number: WO 2004/101590

(56) References cited:
- US-A- 4 180 654
- US-A- 4 518 590

## Description

The present invention relates to novel semi-synthetic macrolides having antimicrobial activity, in particular antibacterial activity. More particularly, the invention relates to 15-membered macrolides substituted at the 4" position, to processes for their preparation, to compositions containing them and to their use in medicine.

Macrolide antibacterial agents are known to be useful in the treatment or prevention of bacterial infections (see, i.e., US 4, 518 590 or 4, 180, 654). However, the emergence of macrolide-resistant bacterial strains has resulted in the need to develop new macrolide compounds.

According to the present invention, we have now found novel 15-membered macrolides substituted at the 4" position which have antimicrobial activity.

Thus, the present invention provides compounds of general formula (I) 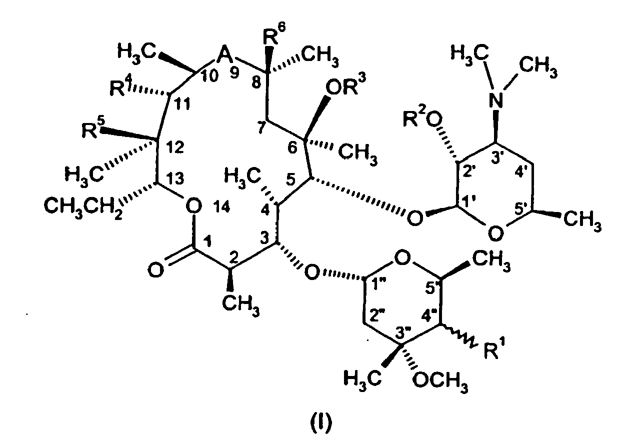 wherein
A is a bivalent radical selected from -C(O)NH-, -NHC(O)-, -N(R⁷)-CH₂- and -CH₂-N(R⁷)-;
R¹ is -NHC(O)(CH₂)_{d}XR⁸;
R² is hydrogen;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R⁹,
R⁵ is hydroxy, or
R⁴ and R⁵ taken together with the intervening atoms form a cyclic group having the following structure: 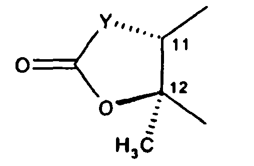 wherein Y is a bivalent radical selected from -CH₂-, -CH(CN)-, -O-, -N(R¹⁰)- and - CH(SR¹⁰)-, with proviso that when A is -NHC(O)-, -N(R⁷)-CH₂- or -CH₂-N(R⁷)-, Y is -O-;
R⁶ is hydrogen or fluorine;
R⁷ is hydrogen or C₁₋₆alkyl;
R⁸ is a heterocyclic group having the following structure: 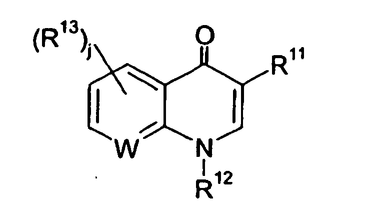 or 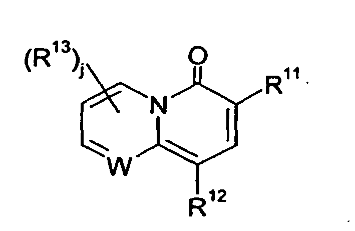
R⁹ is hydrogen or C₁₋₆alkyl;
R¹⁰ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹¹ is hydrogen, -C(O)OR¹⁴, -C(O)NHR¹⁴, -C(O)CH₂NO₂ or -C(O)CH₂SO₂R⁷;
R¹² is hydrogen, C₁₋₄alkyl optionally substituted by hydroxy or C₁₋₄alkoxy, C₃₋₇cycloalkyl, or optionally substituted phenyl or benzyl;
R¹³ is halogen, C₁₋₄alkyl, C₁₋₄thioalkyl, C₁₋₄alkoxy, -NH₂, -NH(C₁₋₄alkyl) or -N(C₁₋₄alkyl)₂;
R¹⁴ is hydrogen,
   C₁₋₆alkyl optionally substituted by up to three groups independently selected from halogen, cyano, C₁₋₄alkoxy optionally substituted by phenyl or C₁₋₄alkoxy, - C(O)C₁₋₆alkyl, -C(O)OC₁₋₆alkyl, -OC(O)C₁₋₆alkyl, -OC(O)OC₁₋₆alkyl, - C(O)NR¹⁷R¹⁸, -NR¹⁷R¹⁸ and phenyl optionally substituted by nitro or -C(O)OC₁₋₆alkyl,
   -(CH₂)_{w}C₃₋₇cycloalkyl,
   -(CH₂)_{w}heterocyclyl,
   -(CH₂)_{w}heteroaryl,
   -(CH₂)_{w}aryl.
   C₃₋₆alkenyl, or
   C₃₋₆alkynyl;
R¹⁵ is hydrogen, C₁₋₄alkyl, C₃₋₇cycloalkyl, optionally substituted phenyl or benzyl, acetyl or benzoyl;
R¹⁶ is hydrogen or R¹³, or R¹⁶ and R¹² are linked to form the bivalent radical -O(CH₂)₂- or -(CH₂)ₜ-;
R¹⁷ and R¹⁸ are each independently hydrogen or C₁₋₆alkyl optionally substituted by phenyl or -C(O)OC₁₋₆alkyl, or
R¹⁷ and R¹⁸, together with the nitrogen atom to which they are bound, form a 5 or 6 membered heterocyclic group optionally containing one additional heteroatom selected from oxygen, nitrogen and sulfur;
X is -U(CH₂)ᵥB-, -U(CH₂)ᵥ- or a group selected from: 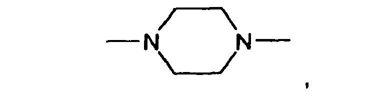 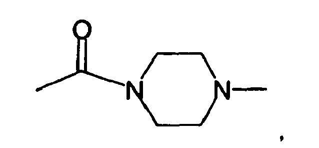 and 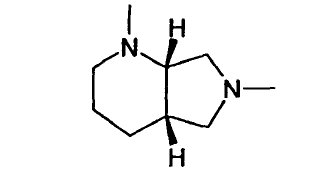
U and B are independently a divalent radical selected from -N(R¹⁵)-, -O-, -S(O)_{z}-, - N(R¹⁵)C(O)-, -C(O)N(R¹⁵)- and -N[C(O)R¹⁵]-;
W is -C(R¹⁶)- or a nitrogen atom;
d is 0 or an integer from 1 to 5;
e is an integer from 2 to 4;
j and z are each independently integers from 0 to 2;
w is an integer from 0 to 4;
t is 2 or 3;
v is an integer from 1 to 8;
and pharmaceutically acceptable derivatives thereof.

According to a further embodiment the present invention provides compounds of general formula (IA) 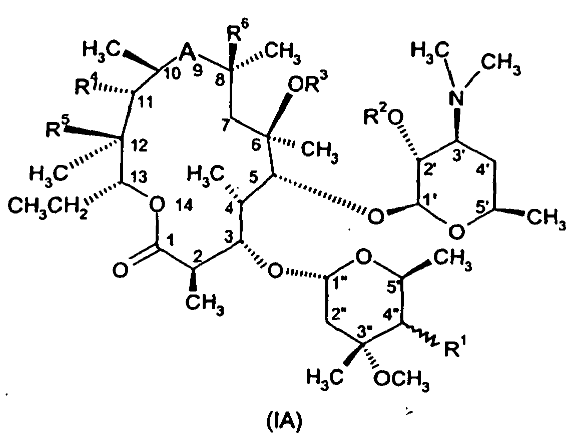 wherein
A is a bivalent radical selected from -C(O)NH-, -NHC(O)-, -N(R⁷)-CH₂- and -CH₂-N(R⁷)-;
R¹ is -NHC(O)(CH₂)_{d}XR⁸;
R² is hydrogen;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R⁹,
R⁵ is hydroxy, or
R⁴ and R⁵ taken together with the intervening atoms form a cyclic group having the following structure: 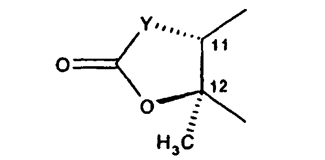 wherein Y is a bivalent radical selected from -CH₂-, -CH(CN)-, -O-, -N(R¹⁰)- and - CH(SR¹⁰)-, with proviso that when A is -NHC(O)-, -N(R⁷)-CH₂- or -CH₂-N(R⁷)-, Y is -O-;
R⁶ is hydrogen or fluorine;
R⁷ is hydrogen or C₁₋₆alkyl;
R⁸ is a heterocyclic group having the following structure: 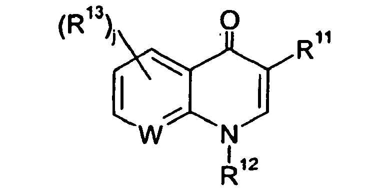 or 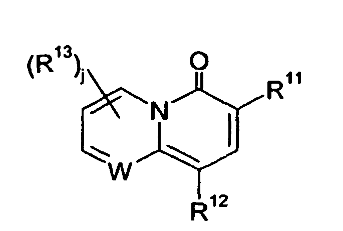
R⁹ is hydrogen or C₁₋₆alkyl;
R¹⁰ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹¹ is hydrogen, -C(O)OR¹⁴, -C(O)NHR¹⁴ or-C(O)CH₂NO₂;
R¹² is hydrogen, C₁₋₄alkyl optionally substituted by hydroxy or C₁₋₄alkoxy, C₃₋₇cycloalkyl, or optionally substituted phenyl or benzyl;
R¹³ is halogen, C₁₋₄alkyl, C₁₋₄thioalkyl, C₁₋₄alkoxy, -NH₂, -NH(C₁₋₄alkyl) or -N(C₁₋₄alkyl)₂;
R¹⁴ is hydrogen or C₁₋₆alkyl optionally substituted by up to three groups independently selected from halogen, C₁₋₄alkoxy, -OC(O)C₁₋₆alkyl and -OC(O)OC₁₋₆alkyl;
R¹⁵ is hydrogen, C₁₋₄alkyl, C₃₋₇cycloalkyl, optionally substituted phenyl or benzyl, acetyl or benzoyl;
R¹⁶ is hydrogen or R¹³, or R¹⁶ and R¹² are linked to form the bivalent radical -O(CH₂)₂- or -(CH₂)ₜ-;
X is -U(CH₂)ᵥB-, -U(CH₂)ᵥ- or a group selected from: 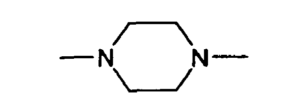 and 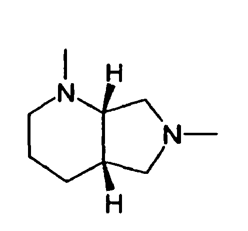
U and B are independently a divalent radical selected from -N(R¹⁵)-, -O-, -S(O)_{z}-, - N(R¹⁵)C(O)-, -C(O)N(R¹⁵)- and -N[C(O)R¹⁵]-;
W is -C(R¹⁶)- or a nitrogen atom;
d is 0 or an integer from 1 to 5;
e is an integer from 2 to 4;
j and z are each independently integers from 0 to 2;
t is 2 or 3;
v is an integer from 2 to 8;
and pharmaceutically acceptable derivatives thereof.

The term "pharmaceutically acceptable" as used herein means a compound which is suitable for pharmaceutical use. Salts and solvates of compounds of the invention which are suitable for use in medicine are those wherein the counterion or associated solvent is pharmaceutically acceptable. However, salts and solvates having non-pharmaceutically acceptable counterions or associated solvents are within the scope of the present invention, for example, for use as intermediates in the preparation of other compounds of the invention and their pharmaceutically acceptable salts and solvates.

The term "pharmaceutically acceptable derivative" as used herein means any pharmaceutically acceptable salt, solvate or prodrug, e.g. ester, of a compound of the invention, which upon administration to the recipient is capable of providing (directly or indirectly) a compound of the invention, or an active metabolite or residue thereof. Such derivatives are recognizable to those skilled in the art, without undue experimentation. Nevertheless, reference is made to the teaching of Burger's Medicinal Chemistry and Drug Discovery, 5th Edition, Vol 1: Principles and Practice, which is incorporated herein by reference to the extent of teaching such derivatives. Preferred pharmaceutically acceptable derivatives are salts, solvates, esters, carbamates and phosphate esters. Particularly preferred pharmaceutically acceptable derivatives are salts, solvates and esters. Most preferred pharmaceutically acceptable derivatives are salts and esters, in particular salts.

The compounds of the present invention may be in the form of and/or may be administered as a pharmaceutically acceptable salt. For a review on suitable salts see Berge et al., J. Pharm. Sci., 1977, 66, 1-19.

Typically, a pharmaceutical acceptable salt may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula (I) and the resulting mixture evaporated to dryness (lyophilised) to obtain the acid addition salt as a solid. Alternatively, a compound of formula (I) may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Suitable addition salts are formed from inorganic or organic acids which form non-toxic salts and examples are hydrochloride, hydrobromide, hydroiodide, sulphate, bisulphate, nitrate, phosphate, hydrogen phosphate, acetate, trifluoroacetate, maleate, malate, fumarate, lactate, tartrate, citrate, formate, gluconate, succinate, pyruvate, oxalate, oxaloacetate, trifluoroacetate, saccharate, benzoate, alkyl or aryl sulphonates (eg methanesulphonate, ethanesulphonate, benzenesulphonate or p-toluenesulphonate) and isethionate. Typical examples include trifluoroacetate and formate salts, for example the bis or tris trifluoroacetate salts and the mono or diformate salts.

Pharmaceutically acceptable base salts include ammonium salts, alkali metal salts such as those of sodium and potassium, alkaline earth metal salts such as those of calcium and magnesium and salts with organic bases, including salts of primary, secondary and tertiary amines, such as isopropylamine, diethylamine, ethanolamine, trimethylamine, dicyclohexyl amine and N-methyl-D-glucamine.

Compounds of the invention may have both a basic and an acidic centre may therefore be in the form of zwitterions.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula (I) may form solvates (e.g. hydrates) and the invention also includes all such solvates.

The term "prodrug" as used herein means a compound which is converted within the body, e.g. by hydrolysis in the blood, into its active form that has medical effects. Pharmaceutically acceptable prodrugs are described in T. Higuchi and V. Stella, "Prodrugs as Novel Delivery Systems", Vol. 14 of the A.C.S. Symposium Series, Edward B. Roche, ed., "Bioreversible Carriers in Drug Design", American Pharmaceutical Association and Pergamon Press, 1987, and in D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

Prodrugs are any covalently bonded carriers that release a compound of structure (I) *in vivo* when such prodrug is administered to a patient. Prodrugs are generally prepared by modifying functional groups in a way such that the modification is cleaved, either by routine manipulation or *in vivo,* yielding the parent compound. Prodrugs include, for example, compounds of this invention wherein hydroxy, amine or sulfhydryl groups are bonded to any group that, when administered to a patient, cleaves to form the hydroxy, amine or sulfhydryl groups. Thus, representative examples of prodrugs include (but are not limited to) acetate, formate and benzoate derivatives of alcohol, sulfhydryl and amine functional groups of the compounds of structure (I). Further, in the case of a carboxylic acid (-COOH), esters may be employed, such as methyl esters, ethyl esters, and the like. Esters may be active in their own right and/or be hydrolysable under *in vivo* conditions in the human body. Suitable pharmaceutically acceptable *in vivo* hydrolysable ester groups include those which break down readily in the human body to leave the parent acid or its salt.

References hereinafter to a compound according to the invention include both compounds of formula (I) and their pharmaceutically acceptable derivatives.

With regard to stereoisomers, the compounds of structure (I) have more than one asymmetric carbon atom. In the general formula (I) as drawn, the solid wedge shaped bond indicates that the bond is above the plane of the paper. The broken bond indicates that the bond is below the plane of the paper. The wavy bond ( 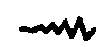 ) indicates that the bond can be either above or below the plane of the paper. Thus, the present invention includes both epimers at the 4"-carbon.

It will be appreciated that the substituents on the macrolide may also have one or more asymmetric carbon atoms. Thus, the compounds of structure (I) may occur as individual enantiomers or diastereomers. All such isomeric forms are included within the present invention, including mixtures thereof.

Where a compound of the invention contains an alkenyl group, cis (Z) and trans (E) isomerism may also occur. The present invention includes the individual stereoisomers of the compound of the invention and, where appropriate, the individual tautomeric forms thereof, together with mixtures thereof.

Separation of diastereoisomers or cis and trans isomers may be achieved by conventional techniques, e.g. by fractional crystallisation, chromatography or H.P.L.C. A stereoisomeric mixture of the agent may also be prepared from a corresponding optically pure intermediate or by resolution, such as H.P.L.C., of the corresponding mixture using a suitable chiral support or by fractional crystallisation of the diastereoisomeric salts formed by reaction of the corresponding mixture with a suitable optically active acid or base, as appropriate.

The compounds of structure (I) may be in crystalline or amorphous form. Furthermore, some of the crystalline forms of the compounds of structure (I) may exist as polymorphs, which are included in the present invention.

R⁶ is hydrogen or fluorine. However, it will be appreciated that when A is -C(O)NH- or - CH₂-N(R⁷)-, R⁶ is hydrogen.

When R⁸ is a heterocyclic group having the following structure: 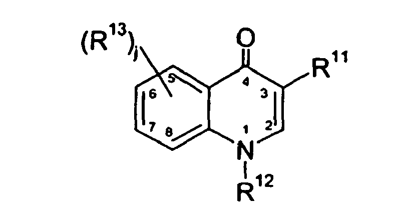 said heterocyclic is linked in the 5, 6, 7 or 8 position to the X group as above defined. In one embodiment, the heterocyclic is linked in the 6 or 7 position. In another embodiment, the heterocyclic is linked in the 5 or 8 position. When present, the R¹³ group or groups may be attached at any position on the ring. In one embodiment, an R¹³ group is attached at the 7 position.

When R⁸ is a heterocyclic group having the following structure: 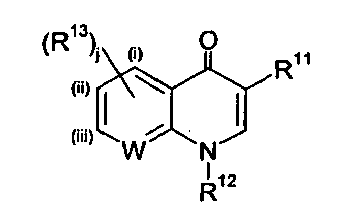 wherein W is -C(R¹⁶)- where R¹⁶ is R¹³ or R¹⁶ and R¹² are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R⁸ is a heterocyclic group having the following structure: 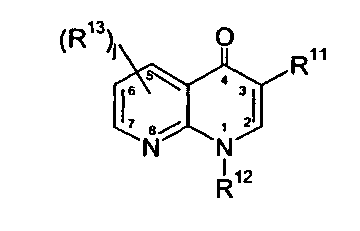 said heterocyclic is linked in the 5, 6 or 7 position to the X group as defined above. In one embodiment, the heterocyclic is linked in the 6 or 7 position. In another embodiment, the heterocyclic is linked in the 5 position.

When R⁸ is a heterocyclic group having the following structure: 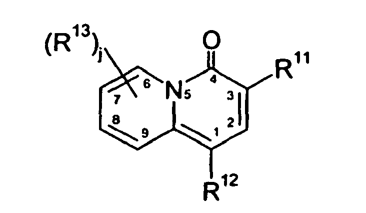 said heterocyclic is linked in the 6, 7, 8 or 9 position to the X group as above defined. In one embodiment, the heterocyclic is linked in the 7 or 8 position. In another embodiment, the heterocyclic is linked in the 6 or 9 position.

When R⁸ is a heterocyclic group having the following structure: 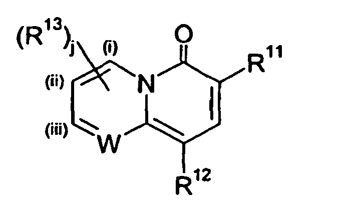 wherein W is -C(R¹⁶)- where R¹⁶ is R¹³ or R¹⁶ and R¹² are linked to form the bivalent radical -O(CH₂)₂- or -(CH₂)ₜ-, said heterocyclic is linked in the (i), (ii) or (iii) position to the X group as above defined. In one embodiment, the heterocyclic is linked in the (i) position. In another embodiment, the heterocyclic is linked in the (ii) or (iii) position.

When R⁸ is a heterocyclic group having the following structure: 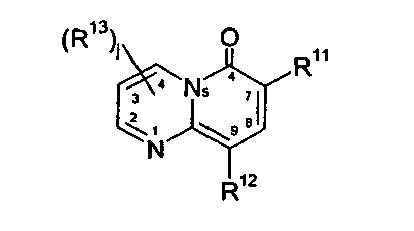 said heterocyclic is linked in the 2, 3 or 4 position to the X group as above defined. In one embodiment, the heterocyclic is linked in the 2 or 3 position. In another embodiment, the heterocyclic is linked in the 4 position.

The term "alkyl" as used herein as a group or a part of a group refers to a straight or branched hydrocarbon chain containing the specified number of carbon atoms. For example, C₁₋₁₀alkyl means a straight or branched alkyl containing at least 1, and at most 10, carbon atoms. Examples of "alkyl" as used herein include, but are not limited to, methyl, ethyl, n-propyl, n-butyl, n-pentyl, isobutyl, isopropyl, t-butyl, hexyl, heptyl, octyl, nonyl and decyl. A C₁₋₄alkyl group is preferred, for example methyl, ethyl, n-propyl, isopropyl, n-butyl, isobutyl or t-butyl.

The term "C₃₋₇cycloalkyl" group as used herein refers to a non-aromatic monocyclic hydrocarbon ring of 3 to 7 carbon atoms such as, for example, cyclopropyl, cyclobutyl, cyclopentyl, cyclohexyl or cycloheptyl.

The term "alkoxy" as used herein refers to a straight or branched chain alkoxy group containing the specified number of carbon atoms. For example, C₁₋₆alkoxy means a straight or branched alkoxy containing at least 1, and at most 6, carbon atoms. Examples of "alkoxy" as used herein include, but are not limited to, methoxy, ethoxy, propoxy, prop-2-oxy, butoxy, but-2-oxy, 2-methylprop-1-oxy, 2-methylprop-2-oxy, pentoxy and hexyloxy. A C₁₋₄alkoxy group is preferred, for example methoxy, ethoxy, propoxy, prop-2-oxy, butoxy, but-2-oxy or 2-methylprop-2-oxy.

The term "alkenyl" as used herein as a group or a part of a group refers to a straight or branched hydrocarbon chain containing the specified number of carbon atoms and containing at least one double bond. For example, the term "C₂₋₆alkenyl" means a straight or branched alkenyl containing at least 2, and at most 6, carbon atoms and containing at least one double bond. Similarly, the term "C₃₋₆alkenyl" means a straight or branched alkenyl containing at least 3, and at most 6, carbon atoms and containing at least one double bond. Examples of "alkenyl" as used herein include, but are not limited to, ethenyl, 2-propenyl, 3-butenyl, 2-butenyl, 2-pentenyl, 3-pentenyl, 3-methyl-2-butenyl, 3-methylbut-2-enyl, 3-hexenyl and 1,1-dimethylbut-2-enyl. It will be appreciated that in groups of the form -O-C₂₋₆alkenyl, the double bond is preferably not adjacent to the oxygen.

The term "alkynyl" as used herein as a group or a part of a group refers to a straight or branched hydrocarbon chain containing the specified number of carbon atoms and containing at least one triple bond. For example, the term "C₃₋₆alkenyl" means a straight or branched alkynyl containing at least 3, and at most 6, carbon atoms containing at least one triple bond. Examples of "alkynyl" as used herein include, but are not limited to, propynyl, 1-butynyl, 2-butynyl, 1-pentynyl and 3-methyl-1-butynyl.

The term "aryl" as used herein refers to an aromatic carbocyclic moiety such as phenyl, biphenyl or naphthyl.

The term "heteroaryl" as used herein, unless otherwise defined, refers to an aromatic heterocycle of 5 to 10 members, having at least one heteroatom selected from nitrogen, oxygen and sulfur, and containing at least 1 carbon atom, including both mono and bicyclic ring systems. Examples of heteroaryl rings include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, thiadiazolyl, pyridyl, pyridazinyl, pyrazinyl, pyrimidinyl, triazinyl, quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl and benzothiophenyl.

The term "5 or 6 membered heteroaryl" as used herein as a group or a part of a group refers to a monocyclic 5 or 6 membered aromatic heterocycle containing at least one heteroatom independently selected from oxygen, nitrogen and sulfur. Examples include, but are not limited to, furanyl, thiophenyl, pyrrolyl, pyrazolyl, imidazolyl, oxazolyl, isoxazolyl, thiazolyl, isothiazolyl, triazolyl, oxadiazolyl, tetrazolyl, pyridyl, pyridazinyl, pyrazinyl, pyrimidinyl and triazinyl.

The term "9 to 10 membered fused bicyclic heteroaryl" as used herein as a group or a part of a group refers to quinolinyl, isoquinolinyl, 1,2,3,4-tetrahydroisoquinolinyl, benzofuranyl, benzimidazolyl, benzothienyl, benzoxazolyl, 1,3-benzodioxazolyl, indolyl, benzothiazolyl, furylpyridine, oxazolopyridyl or benzothiophenyl.

The term "heterocyclyl" as used herein, unless otherwise defined, refers to a monocyclic or bicyclic three- to ten-membered saturated or non-aromatic, unsaturated hydrocarbon ring containing at least one heteroatom selected from oxygen, nitrogen and sulfur. Preferably, the heterocyclyl ring has five or six ring atoms. Examples of heterocyclyl groups include, but are not limited to, pyrrolidinyl, tetrahydrofuranyl, tetrahydrothiophenyl, imidazolidinyl, pyrazolidinyl, piperidyl, piperazinyl, morpholino, tetrahydropyranyl and thiomorpholino.

The term "halogen" refers to a fluorine, chlorine, bromine or iodine atom.

The terms "optionally substituted phenyl", "optionally substituted phenyl or benzyl", "optionally substituted 5 or 6 membered heteroaryl" or "optionally substituted 9 to 10 membered fused bicyclic heteroaryl" as used herein refer to a group which is substituted by 1 to 3 groups selected from halogen, C₁₋₄alkyl, C₁₋₄alkoxy, hydroxy, nitro, cyano, amino, C₁₋₄alkylamino or diC₁₋₄alkylamino, phenyl and 5 or 6 membered heteroaryl.

In one embodiment, A is -N(R⁷)-CH₂- or -CH₂-N(R⁷)-. A representative example of A is - N(R⁷)-CH₂-.

A representative example of R² is hydrogen.

In one embodiment, R³ is hydrogen or C₁₋₄alkyl. A representative example of R³ is hydrogen.

In one embodiment, R⁴ and R⁵ are hydroxy, or R⁴ and R⁵ taken together with the intervening atoms form a cyclic group having the following structure: 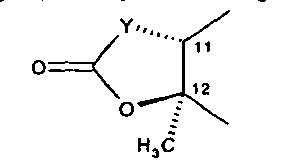 wherein Y is the bivalent radical -O-. A representative example of R⁴ and R⁵ is hydroxy. Alternatively, R⁴ and R⁵ taken together with the intervening atoms form a cyclic group having the following structure: 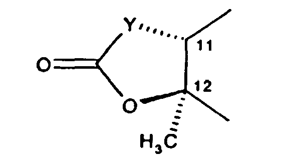 wherein Y is a bivalent radical selected from -O-.

A representative example of R⁶ is hydrogen.

A representative example of R⁷ is C₁₋₆alkyl, for example C₁₋₄alkyl, in particular methyl.

Representative examples of R⁸ include heterocyclic groups having the following structure: 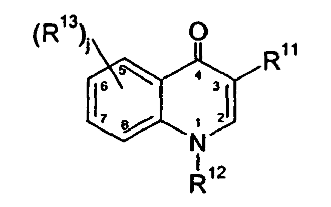 wherein the heterocyclic is linked in the 6 or 7 position to the X group as above defined. In particular, the heterocyclic is linked in the 6 position.

In one embodiment, R¹⁰ is hydrogen or C₁₋₄alkyl substituted by a group selected from optionally substituted phenyl, optionally substituted 5 or 6 membered heteroaryl and optionally substituted 9 to 10 membered fused bicyclic heteroaryl.

In one embodiment, R¹¹ is hydrogen, -C(O)OR¹⁴, -C(O)NHR¹⁴ or -C(O)CH₂NO₂. In another embodiment, R¹¹ is -C(O)OR¹⁴, -C(O)NHR¹⁴ or -C(O)CH₂NO₂. In a further embodiment, R¹¹ is -C(O)OR¹⁴. A representative example of R¹¹ is -C(O)OR¹⁴, wherein R¹⁴ is hydrogen. A further representative example of R¹¹ is -C(O)OR¹⁴, wherein R¹⁴ is C₁₋₄alkyl.

A representative example of R¹² is C₃₋₇cycloalkyl, in particular cyclopropyl.

A representative example of R¹³ is halogen, in particular chlorine.

In one embodiment, R¹⁴ is hydrogen or C₁₋₆alkyl optionally substituted by up to three groups independently selected from halogen, C₁₋₄alkoxy, -OC(O)C₁₋₆alkyl and - OC(O)OC₁₋₆alkyl. Representative examples of R¹⁴ include hydrogen and C₁₋₄alkyl, in particular hydrogen and methyl.

In one embodiment, R¹⁵ is hydrogen or C₁₋₄alkyl. A representative example of R¹⁵ is hydrogen. A further representative example of R¹⁵ is methyl.

A representative example of R¹⁶ is hydrogen.

In one embodiment, X is -U(CH₂)ᵥB-, -U(CH₂)ᵥ- or a group selected from: 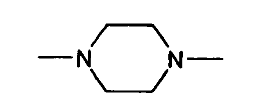 and

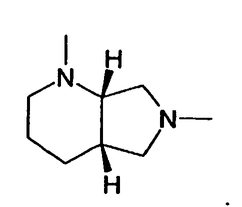

A representative example of X is -U(CH₂)ᵥB-.

Representative examples of U and B include the divalent radicals -N(R¹⁵)- and -O-. In particular, U is -O- and B is a divalent radical selected from -N(R¹⁵)- and -O-. Alternatively, U and B are each independently the divalent radical -N(R¹⁵)-.

A representative example of Y is the bivalent radical -O-.

A representative example of d is 1 to 3, for example 2.

In one embodiment, v is an integer from 2 to 8. A representative example of v is 2 to 4, for example 2.

In one embodiment, j is 0 or 1. A representative example of j is 1. A further representative example of j is 0.

It is to be understood that the present invention covers all combinations of particular and preferred groups described hereinabove. It is also to be understood that the present invention encompasses compounds of formula (I) in which a particular group or parameter, for example R⁷, R¹³, R¹⁵, R¹⁷, R¹⁸ and z may occur more than once. In such compounds it will be appreciated that each group or parameter is independently selected from the values listed.

Particularly preferred compounds of the invention are:
4"-(S)-{3-[2-(3-carboxy-7-chloro-1-cyclopropyl-4-oxo-1,4-dihydro-quinolin-6-ylamino)-ethoxy]-propionylamino}-4"-deoxyazithromycin;
4"-(R)-{3-[2-(3-carboxy-7-chloro-1-cyclopropyl-4-oxo-1,4-dihydro-quinolin-6-ylamino)-ethoxy]-propionylamino}-4"-deoxyazithromycin;
4"-(S)-{3-[2-(3-carboxy-7-chloro-1-cyclopropyl-4-oxo-1,4-dihydro-quinolin-6-yloxy)-ethoxy]-propionylamino}-4"-deoxyazithromycin;
and pharmaceutically acceptable derivatives thereof.

Further particularly preferred compounds of the invention are:
4"-(S)-{3-[2-(3-carboxy-1-cyclopropyl-4-oxo-1,4-dihydro-quinolin-6-yloxy)-ethoxy]-propionylamino}-4"-deoxyazithromycin 11,12-cyclic carbonate;
4"-(3-{[2-(7-chloro-1-cyclopropyl-3-methoxycarbonyl-4-oxo-1,4-dihydro-quinolin-6-ylamino)-ethyl]-methyl-amino}-propionylamino)-4"-deoxyazithromycin;
and pharmaceutically acceptable derivatives thereof.

Compounds according to the invention also exhibit a broad spectrum of antimicrobial activity, in particular antibacterial activity, against a wide range of clinical pathogenic microorganisms. Using a standard microtiter broth serial dilution test, compounds of the invention have been found to exhibit useful levels of activity against a wide range of pathogenic microorganisims. In particular, the compounds of the invention may be active against strains of Staphylococcus aureus, Streptopococcus pneumoniae, Moraxella catarrhalis, Streptococcus pyogenes, Haemophilus influenzae, Enterococcus faecalis, Chlamydia pneumoniae, Mycoplasma pneumoniae and Legionella pneumophila. The compounds of the invention may also be active against resistant strains, for example erythromycin resistant strains. In particular, the compounds of the invention may be active against erythromycin resistant strains of Streptococcus pneumoniae, Streptococcus pyogenes and Staphylococcus aureus.

The compounds of the invention may therefore be used for treating a variety of diseases caused by pathogenic microorganisms, in particular bacteria, in human beings and animals. It will be appreciated that reference to treatment includes acute treatment or prophylaxis as well as the alleviation of established symptoms.

Thus, according to another aspect of the present invention we provide a compound of formula (I) or a pharmaceutically acceptable derivative thereof for use in therapy.

According to a further aspect of the invention we provide a compound of formula (I) or a pharmaceutically acceptable derivative thereof for use in the therapy or prophylaxis of systemic or topical microbial infections in a human or animal subject.

According to a further aspect of the invention we provide the use of a compound of formula (I) or a pharmaceutically acceptable derivative thereof in the manufacture of a medicament for use in the treatment or prophylaxis of systemic or topical microbial infections in a human or animal body.

According to a yet further aspect of the invention we provide a method of treatment of the human or non-human animal body to combat microbial infections comprising administration to a body in need of such treatment of an effective amount of a compound of formula (I) or a pharmaceutically acceptable derivative thereof.

While it is possible that, for use in therapy, a compound of the invention may be administered as the raw chemical it is preferable to present the active ingredient as a pharmaceutical formulation eg when the agent is in admixture with a suitable pharmaceutical excipient, diluent or carrier selected with regard to the intended route of administration and standard pharmaceutical practice.

Accordingly, in one aspect, the present invention provides a pharmaceutical composition or formulation comprising at least one compound of the invention or a pharmaceutically acceptable derivative thereof in association with a pharmaceutically acceptable excipient, diluent and/or carrier. The excipient, diluent and/or carrier must be "acceptable" in the sense of being compatible with the other ingredients of the formulation and not deleterious to the recipient thereof.

In another aspect, the invention provides a pharmaceutical composition comprising, as active ingredient, at least one compound of the invention or a pharmaceutically acceptable derivative thereof in association with a pharmaceutically acceptable excipient, diluent and/or carrier for use in therapy, and in particular, in the treatment of human or animal subjects suffering from a condition susceptible to amelioration by an antimicrobial compound.

In another aspect, the invention provides a pharmaceutical composition comprising a therapeutically effective amount of the compounds of the present invention and a pharmaceutically acceptable excipient, diluent and/or carrier (including combinations thereof).

There is further provided by the present invention a process of preparing a pharmaceutical composition, which process comprises mixing at least one compound of the invention or a pharmaceutically acceptable derivative thereof, together with a pharmaceutically acceptable excipient, diluent and/or carrier.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable excipients, diluents and/or carriers. Acceptable excipients, diluents and carriers for therapetic use are well known in the pharmaceutical art, and are described, for example, in Remington's Pharmaceutical Sciences, Mack Publishing Co. (A. R. Gennaro edit. 1985). The choice of pharmaceutical excipient, diluent and/or carrier can be selected with regard to the intended route of administration and standard pharmaceutical practice. The pharmaceutical compositions may comprise as - or in addition to - the excipient, diluent and/or carrier any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

For some embodiments, the agents of the present invention may also be used in combination with a cyclodextrin. Cyclodextrins are known to form inclusion and non-inclusion complexes with drug molecules. Formation of a drug-cyclodextrin complex may modify the solubility, dissolution rate, bioavailability and/or stability property of a drug molecule. Drug-cyclodextrin complexes are generally useful for most dosage forms and administration routes. As an alternative to direct complexation with the drug the cyclodextrin may be used as an auxiliary additive, e. g. as a carrier, diluent or solubiliser. Alpha-, beta- and gamma-cyclodextrins are most commonly used and suitable examples are described in WO 91/11172, WO 94/02518 and WO 98/55148.

The compounds of the invention may be milled using known milling procedures such as wet milling to obtain a particle size appropriate for tablet formation and for other formulation types. Finely divided (nanoparticulate) preparations of the compounds of the invention may be prepared by processes known in the art, for example see International Patent Application No. WO 02/00196 (SmithKline Beecham).

The routes for administration (delivery) include, but are not limited to, one or more of: oral (e. g. as a tablet, capsule, or as an ingestable solution), topical, mucosal (e. g. as a nasal spray or aerosol for inhalation), nasal, parenteral (e. g. by an injectable form), gastrointestinal, intraspinal, intraperitoneal, intramuscular, intravenous, intrauterine, intraocular, intradermal, intracranial, intratracheal, intravaginal, intracerebroventricular, intracerebral, subcutaneous, ophthalmic (including intravitreal or intracameral), transdermal, rectal, buccal, epidural and sublingual.

There may be different composition/formulation requirements depending on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

It is to be understood that not all of the compounds need be administered by the same route. Likewise, if the composition comprises more than one active component, then those components may be administered by different routes.

The compositions of the invention include those in a form especially formulated for parenteral, oral, buccal, rectal, topical, implant, ophthalmic, nasal or genito-urinary use. For some applications, the agents of the present invention are delivered systemically (such as orally, buccally, sublingually), more preferably orally. Hence, preferably the agent is in a form that is suitable for oral delivery.

If the compound of the present invention is administered parenterally, then examples of such administration include one or more of: intravenously, intraarterially, intraperitoneally, intrathecally, intraventricularly, intraurethrally, intrastemally, intracranially, intramuscularly or subcutaneously administering the agent; and/or by using infusion techniques.

For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

The compounds according to the invention may be formulated for use in human or veterinary medicine by injection (e.g. by intravenous bolus injection or infusion or via intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilising, solubilising and/or dispersing agents. Alternatively the active ingredient may be in sterile powder form for reconstitution with a suitable vehicle, e.g. sterile, pyrogen-free water, before use.

The compounds of the invention can be administered (e. g. orally or topically) in the form of tablets, capsules, ovules, elixirs, solutions or suspensions, which may contain flavouring or colouring agents, for immediate-, delayed-, modified-, sustained-, pulsed-or controlled-release applications.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably com, potato or tapioca starch), sodium starch glycollate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may also be administered orally in veterinary medicine in the form of a liquid drench such as a solution, suspension or dispersion of the active ingredient together with a pharmaceutically acceptable carrier or excipient.

The compounds of the invention may also, for example, be formulated as suppositories e.g. containing conventional suppository bases for use in human or veterinary medicine or as pessaries e.g. containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops (e.g. eye ear or nose drops) or pour-ons.

For application topically to the skin, the agent of the present invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax and water.

Alternatively, it can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, cetearyl alcohol, 2-octyldodecanol, benzyl alcohol and water.

The compounds may also be dermally or transdermally administered, for example, by use of a skin patch.

For ophthalmic use, the compounds can be formulated as micronised suspensions in isotonic, pH adjusted, sterile saline, or, preferably, as solutions in isotonic, pH adjusted, sterile saline, optionally in combination with a preservative such as a benzylalkonium chloride. Alternatively, they may be formulated in an ointment such as petrolatum.

As indicated, the compound of the present invention can be administered intranasally or by inhalation and is conveniently delivered in the form of a dry powder inhaler or an aerosol spray presentation from a pressurised container, pump, spray or nebuliser with the use of a suitable propellant, e. g. dichlorodifluoromethane, trichlorofluoromethane, dichlorotetrafluoroethane, a hydrofluoroalkane such as 1,1,1,2-tetrafluoroethane (HFA 134AT"') or 1,1,1,2,3,3,3-heptafluoropropane (HFA 227EA), carbon dioxide or other suitable gas. In the case of a pressurised aerosol, the dosage unit may be determined by providing a valve to deliver a metered amount. The pressurised container, pump, spray or nebuliser may contain a solution or suspension of the active compound, e. g. using a mixture of ethanol and the propellant as the solvent, which may additionally contain a lubricant, e. g. sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine via a nebuliser.

The compounds of the invention may also be used in combination with other therapeutic agents. The invention thus provides, in a further aspect, a combination comprising a compound of the invention or a pharmaceutically acceptable derivative thereof together with a further therapeutic agent.

When a compound of the invention or a pharmaceutically acceptable derivative thereof is used in combination with a second therapeutic agent active against the same disease state the dose of each compound may differ from that when the compound is used alone. Appropriate doses will be readily appreciated by those skilled in the art. It will be appreciated that the amount of a compound of the invention required for use in treatment will vary with the nature of the condition being treated and the age and the condition of the patient and will be ultimately at the discretion of the attendant physician or veterinarian. The compounds of the present invention may for example be used for topical administration with other active ingredients such as corticosteroids or antifungals as appropriate.

The combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations by any convenient route.

When administration is sequential, either the compound of the invention or the second therapeutic agent may be administered first. When administration is simultaneous, the combination may be administered either in the same or different pharmaceutical composition.

When combined in the same formulation it will be appreciated that the two compounds must be stable and compatible with each other and the other components of the formulation. When formulated separately they may be provided in any convenient formulation, conveniently in such manner as are known for such compounds in the art.

The compositions may contain from 0.01-99% of the active material. For topical administration, for example, the composition will generally contain from 0.01-10%, more preferably 0.01-1% of the active material.

Typically, a physician will determine the actual dosage which will be most suitable for an individual subject. The specific dose level and frequency of dosage for any particular individual may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy.

For oral and parenteral administration to humans, the daily dosage level of the agent may be in single or divided doses.

For systemic administration the daily dose as employed for adult human treatment it will range from 2-100mg/kg body weight, preferably 5-60mg/kg body weight, which may be administered in 1 to 4 daily doses, for example, depending on the route of administration and the condition of the patient. When the composition comprises dosage units, each unit will preferably contain 200mg to 1g of active ingredient. The duration of treatment will be dictated by the rate of response rather than by arbitrary numbers of days.

Compounds of general formula (I) and salts thereof may be prepared by the general methods outlined hereinafter, said methods constituting a further aspect of the invention. In the following description, the groups R¹ to R¹⁸, A, B, X, Y, U, W, d, e, j, t, v, w and z have the meaning defined for the compounds of formula (I) unless otherwise stated.

The group X^{a}R^{8a} is XR⁸ as defined for formula (I) or a group convertible to XR⁸. Similarly, the group B^{a}R^{8a} is BR⁸ as defined for formula (I) or a group convertible to BR⁸. Conversion of a group X^{a}R^{8a} or B^{a}R^{8a} to a XR⁸ or BR⁸ group typically arises if a protecting group is needed during the reactions described below. A comprehensive discussion of the ways in which such groups may be protected and methods for cleaving the resulting protected derivatives is given by for example T.W. Greene and P.G.M Wuts in Protective Groups in Organic Synthesis 2nd ed., John Wiley & Son, Inc 1991 and by P.J. Kocienski in Protecting Groups, Georg Thieme Verlag 1994 which are incorporated herein by reference. Examples of suitable amino protecting groups include acyl type protecting groups (e.g. formyl, trifluoroacetyl and acetyl), aromatic urethane type protecting groups (e.g. benzyloxycarbonyl (Cbz) and substituted Cbz, and 9-fluorenylmethoxycarbonyl (Fmoc)), aliphatic urethane protecting groups (e.g. t-butyloxycarbonyl (Boc), isopropyloxycarbonyl and cyclohexyloxycarbonyl) and alkyl type protecting groups (e.g. benzyl, trityl and chlorotrityl). Examples of suitable oxygen protecting groups may include for example alkyl silyl groups, such as trimethylsilyl or tert-butyldimethylsilyl; alkyl ethers such as tetrahydropyranyl or tert-butyl; or esters such as acetate. Hydroxy groups may be protected by reaction of for example acetic anhydride, benzoic anhydride or a trialkylsilyl chloride in an aprotic solvent. Examples of aprotic solvents are dichloromethane, N,N-dimethytformamide, dimethylsuffoxide, tetrahydrofuran and the like.

Compounds of formula (I) wherein d is an integer from 1 to 5 may be prepared by reaction of a 4" amine compound of formula (II) with a carboxylic acid compound of formula (III), or a suitable activated and protected derivative thereof, followed where necessary by subsequent conversion of the X^{a}R^{8a} group to XR⁸.

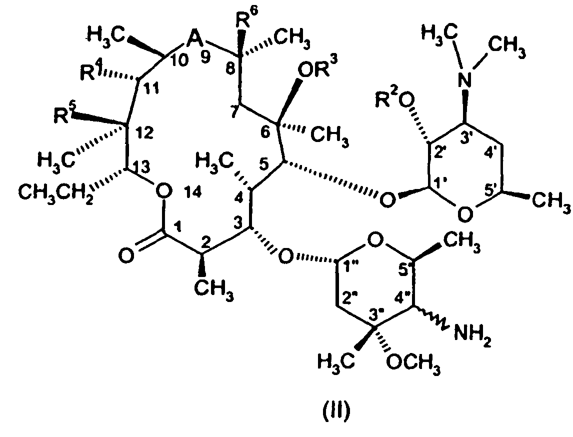

HOC(O)(CH₂)_{d}X^{a}R^{8a} (III)

Suitable activated derivatives of the carboxyl group include the corresponding acyl halide, mixed anhydride or activated ester such as a thioester. The reaction is preferably carried out in a suitable aprotic solvent such as a halohydrocarbon (e.g. dichloromethane) or N,N-dimethylformamide optionally in the presence of a tertiary organic base such as dimethylaminopyridine or triethylamine or in the presence of inorganic base (eg sodium hydroxide) and at a temperature within the range of 0° to 120°C. The compounds of formula (II) and (III) may also be reacted in the presence of a carbodiimide such as dicyclohexylcarbodiimide (DCC).

Compounds of formula (I) wherein d is 0 and U is -C(O)N(R¹⁵)- may be prepared by reaction of the 4" amine of formula (II) with a suitable activated derivative of the carboxylic acid HOC(O)C(O)N(R¹⁵)(CH₂)ᵥB^{a}R^{8a} (IV) followed where necessary by subsequent removal of the hydroxyl protecting group R² and conversion of the B^{a}R^{8a} group to BR⁸.

Compounds of formula (I) wherein d is 0 and U is -NH- may be prepared by reaction of the 4" amine of formula (II) with a suitable activated derivative such as the isocyanate OCN(CH₂)ᵥB^{a}R^{8a}.

Compounds of formula (I) wherein d is 0 and U is -N(R¹⁵)- may be prepared by reaction of the 4" amine of formula (II) with a suitable activated derivative such as the carbamoyl chloride CIC(O)N(R¹⁵)(CH₂)ᵥB^{a}R^{8a}.

Compounds of formula (I) wherein d is 0 and U is -O- may be prepared by reaction of the 4" amine of formula (II) with a suitable activated derivative such as the chloroformate ClOC(O)O(CH₂)ᵥB^{a}R^{15a}.

In a further embodiment of the invention, compounds of formula (I) wherein d is an integer from 1 to 5 and U is a group selected from -N(R¹⁵)- and -S-, may be prepared by reaction of compounds of formula (V) 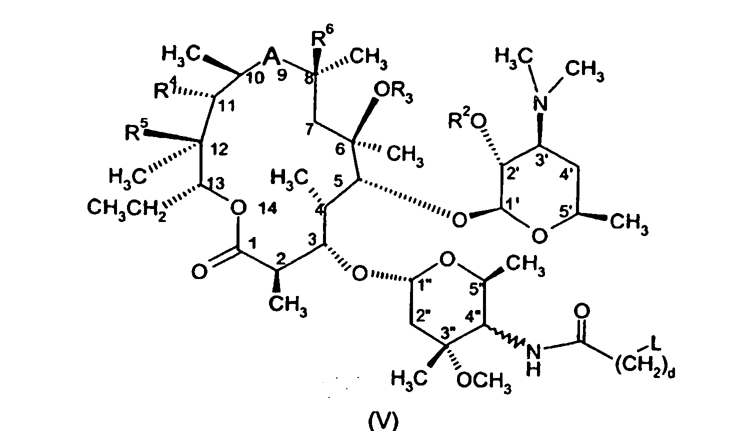 wherein d is an integer from 1 to 5 and L is a suitable leaving group, with X^{a}R^{8a} (VI) in which U is a group selected from -N(R¹⁵)- and -S-. The reaction is preferably carried out in a solvent such as a halohydrocarbon (e.g. dichloromethane), an ether (e.g. tetrahydrofuran or dimethoxyethane), acetonitrile or ethyl acetate and the like, dimethylsulfoxide, N,N-dimethylformamide or 1-methyl-pyrrolidone and in the presence of a base, followed, if desired, by conversion of the X^{a}R^{8a} group to XR⁸. Examples of the bases which may be used include organic bases such as diisopropylethylamine, triethylamine and 1,8-diazabicyclo[5.4.0]undec-7-ene, and inorganic bases such as potassium hydroxide, cesium hydroxide, tetraalkylammonium hydroxide, sodium hydride, potassium hydride and the like. Suitable leaving groups for this reaction include halide (e.g. chloride, bromide or iodide) or a sulfonyloxy group (e.g. tosyloxy or methanesulfonyloxy).

Compounds of formula (V) may be prepared by reaction of a compound of formula (II) with a carboxylic acid HOC(O)(CH₂)_{d}L (VII), wherein L is a suitable leaving group as above defined, or a suitable activated thereof. Suitable activated derivatives of the carboxyl group are those defined above for carboxylic acid (III). The reaction is carried out using the conditions described above for the reaction of a compound of formula (II) with carboxylic acid (III).

Compounds of formula (I) may be converted into other compounds of formula (I). Thus compounds of formula (I) wherein U or B is -S(O)_{z}- and z is 1 or 2 may be prepared by oxidation of the corresponding compound of formula (I) wherein z is 0. The oxidation is preferably carried out using a peracid, e.g. peroxybenzoic acid, followed by treatment with a phosphine, such as triphenylphosphine. The reaction is suitably carried out in an organic solvent such as methylene chloride. Compounds of formula (l) wherein U or B is -N(R¹⁵)-and R¹⁵ is C₁₋₄alkyl can be prepared from compounds wherein R¹⁵ is hydrogen by reductive alkylation.

Compounds of formula (II) wherein A is -C(O)NH- or -NHC(O)-, R⁴ or R⁵ are hydroxy, R³ is hydrogen and R⁶ is hydrogen are known compounds or they may be prepared by analogous methods to those known in the art. Thus they can be prepared according to the procedures described in EP 507595 and EP 503932.

Compounds of formula (II), wherein A is -C(O)NH- or -NHC(O)-, R⁴ or R⁵ are hydroxy and R³ is C₁₋₄alkyl or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in WO 9951616 and WO 0063223.

Compounds of formula (II), wherein A is -C(O)NH-, R⁴ and R⁵ taken together with the intervening atoms form a cyclic group having the following structure:

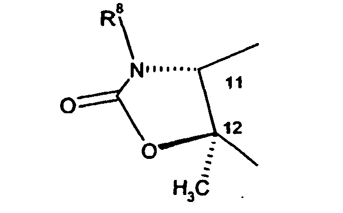

R³ is C₁₋₄alkyl, or C₃₋₆alkenyl optionally substituted by 9 to 10 membered fused bicyclic heteroaryl and R⁶ is hydrogen are known compounds or they may be prepared by analogous methods to those known in the art. Thus they can be prepared according to the procedures described in US 6262030.

Compounds of formula (II), wherein A is -C(O)NH-, -NHC(O)-, -N(CH₃)-CH₂- or -CH₂-N(CH₃)-, R⁴ or R⁵ are hydroxy or R⁴ and R⁵ taken together with the intervening atoms form a cyclic group having the following structure: 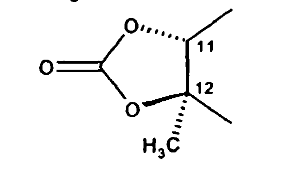 and R⁶ is hydrogen are known compounds or they may be prepared by analogous methods to those known in the art. Thus they can be prepared according to the procedures described in EP 508699 and J.Chem. Res.Synop (1988 pages 152-153), US 6262030.

Compounds of formula (II), wherein A is -C(O)NH-, R⁴ and R⁵ taken together with the intervening atoms form a cyclic group having the following structure: 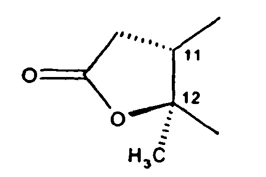 R⁶ is hydrogen and R³ is C₁₋₄ alkyl may be prepared by decarboxylation of a compound of formula (VIII), wherein R¹⁹ is amino protecting group followed, if required, by removal of the protecting group R¹⁹.

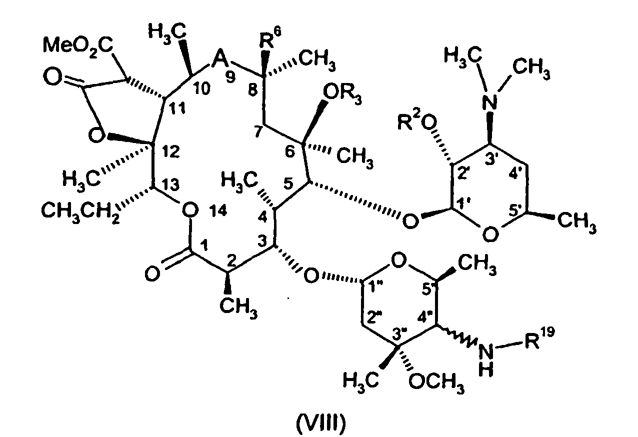

The decarboxylation may be carried out in the presence of a lithium salt such as lithium chloride, preferably in an organic solvent such as dimethylsulfoxide.

Compounds of formula (II), wherein A is -C(O)NH-, R⁴ and R⁵ taken together with the intervening atoms form a cyclic group having the following structure: 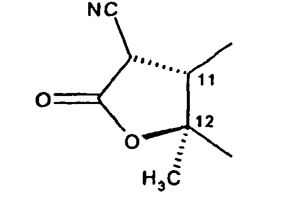 and R₃ is C₁₋₄ alkyl may be prepared according to the procedures described in WO 02/50091 and WO 02/50092.

Compounds of formula (III) wherein X is -U(CH₂)ᵥ- or -U(CH₂)ᵥN(R¹⁵)-, in which U is - N(R¹⁵)-, -O- or -S-, or X is a group selected from: 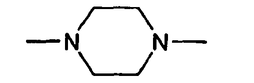 and 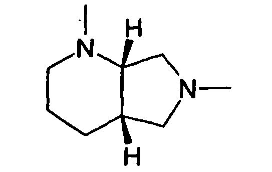 may be prepared by reaction of X^{a}R^{8a} (VI), wherein X has the meaning defined above with R²⁰OC(O)(CH₂)_{d}L (IX) wherein R²⁰ is carboxyl protecting group and L is a suitable leaving group, followed by removal of R²⁰. Suitable R²⁰ carboxyl protecting group include t-butyl, allyl or benzyl.

Compounds of formula (III) may also be prepared by reaction of X^{a}R^{8a} (VI) with acrylonitrile followed by hydrolysis of the nitrile to the acid, or by reaction of X^{a}R^{8a} (VI) with t-butyl acrylate followed by removal of the t-butyl group.

Compounds of formula (VI) wherein X is -U(CH₂)ᵥB- in which B is -N(R¹⁵)-, -O- or -S- or X is a group selected from: 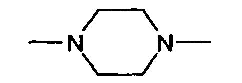 or 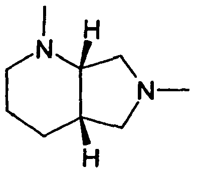 may be prepared by reaction of a compound of formula R^{8a}L (X), wherein L is a suitable leaving group such as chlorine, fluorine or bromine, with a compound of formula - U(CH₂)ᵥB- (X) in which B is -N(R¹⁵)-, -O- or -S-, or with piperazine or with 1H-pyrrolo[3,4-b]pyridine, octahydro.

In order that the invention may be more fully understood the following examples are given by way of illustration only.

The following abbreviations are used in the text: DBU for 1,8-diazabicyclo[5.4.0]undec-7-ene, DCC for dicyclohexylcarbodiimide, DCM for dichloromethane, DMAP for 4-dimethylaminopyridine, DMF for N,N-dimethylformamide, DMS for dimethylsulfide, DMSO for dimethyl sulfoxide, EtOAc for ethyl acetate, EtOH for ethanol, KO^{t}Bu for potassium *tert*-butoxide, MeOH for methanol and i-PrOH for isopropanol.

### Examples

4"-(S) and 4"-(R)-Amino-9a-azithromycin may be prepared by the procedure described ir-EP 508 699. 4"-Keto-9a-azithromycin may be prepared using the Pfitzner-Moffat procedure (J. Am.Chem.Soc.,87, 5670-5678, 1965) at room temperature for 4 hours and deprotecting in MeOH.

### Intermediate 1

### 7-Chloro-1-cyclopropryl-6-(2-hydroxy-ethylamino)-4-oxo-1,4-dihydro-quinoline-3-carboxylic acid (A) and 1-Cyclopropyl-fluoro-7-(2-hydroxy-ethylamino)-4-oxo-1,4-dihydro-quinoline-3-carboxylic acid (B)

To a solution of ethanolamine (55.5 mL) in N-methyl pyrrolidinone (500 mL) at 95°C, 7-chloro-1-cyclopropyl-6-fluoro-4-oxo-1,4-dihydro-quinoline-3-carboxylic acid (50.0 g) was slowly added under vigorous stirring. The temperature was increased to 105 °C and the reaction mixture was stirred at this temperature for 22 hours. The reaction mixture was cooled to about 60 °C and poured into MeOH (800 mL). This mixture was stirred in an ice bath and the precipitate was filtered off and dried affording a mixture of **Intermediate** 1A and **Intermediate 1B** (49 g) in a 1:1 ratio.
**Intermediate 1A:** MS; m/z (ES): 322.99 [MH]⁺
**Intermediate 1B:** MS; m/z (ES): 307.02 [MH]⁺

### Intermediate 2

### 7-Chloro-6-[2-(2-cyano-ethoxy)-ethylamino]-1-cyclopropyl-4-oxo-1,4-dihydroquinoline-3-carboxylic acid (A) and 7-[2-(2-Cyano-ethoxy)-ethylamino]-1-cyclopropyl-6-fluoro-4-oxo-1,4-dihydroquinoline-3-carboxylic acid (B)

A solution of a mixture of **Intermediate 1A** and **Intermediate 1B** (14 g) in acrylonitrile (140 mL) and DBU (14 mL) was stirred at 70 °C for 16 hours. The solvent was evaporated and the residue dissolved in i-PrOH (50 mL). Water (50 mL) was added and the pH value adjusted to 4. The precipitate was filtered and then triturated with methanol. After filtration, 5.35 g of pure **Intermediate 2A** was obtained. The mother liquor was left overnight at 4 °C and 4.4 g of **Intermediate 2B** precipitated.

**Intermediate 2A:** ¹H-NMR (500 MHz, DMSO-d6) δ: 8.56 (s, 1H), 8.23 (s, 1H), 7.40 (s, 1H), 5.93 (t, NH), 3.83 (qv, 1 H), 3.72 (t, 2H), 3.67 (t, 2H), 3.46 (q, 2H), 2.79 (t, 2H), 1.30 (q, 2H), 1.18 (q, 2H). ¹³C-NMR (75 MHz, DMSO-d6) δ: 176.52, 166.09, 145.72, 142.72, 132.17, 126.37, 125.38, 119.15, 118.99, 106.14, 102.76, 67.93, 65.05, 42.40, 35.77. 18.01, 7.32. MS; m/z (ES): 376.02 [MH]⁺

**Intermediate 2B:** ¹H-NMR (500 MHz, DMSO-d6) δ: 8.55 (s, 1H), 7.76 (d, 1H), 7.22 (d, 1H), 3.74 (t, 2H+1H), 3.67 (t, 2H), 3.52 (q, 2H), 2.78 (t, 2H), 1.31 (m, 2H), 1.18 (m, 2H). ¹³C-NMR (75 MHz, DMSO-d6) δ: 175.80, 166.20, 148.12, 146.89, 142.55, 140.30, 119.22, 108.79, 106.10, 96.68, 68.29, 65.17, 42.06, 35.70, 17.99, 7.48. MS; m/z (ES): 360.04 [MH]⁺

### Intermediate 3

### 6-[2-(2-Carboxy-ethoxy)-ethylamino]-7-chloro-1-cyclopropyl-4-oxo-1,4-dihydroquinoline-3-carboxylic acid

A solution of **Intermediate 2A** (4.7 g) in 60 mL conc. H₂SO₄ and 60 mL H₂O was stirred for 20 hours at 75 °C. The reaction mixture was poured into water (150 mL) and the pH value was adjusted to 2. Filtration of the precipitate obtained yielded pure **Intermediate 3** (3.07 g); ¹H-NMR (500 MHz, DMSO-d6) δ: 8.56 (s, 1H), 8.23 (s, 1H), 7.39 (s, 1H), 3.82 (m, 1H), 3.66 (q, 2H+2H), 3.42 (t, 2H), 2.49 (t, 2H), 1.30 (q, 2H), 1.17 (m, 2H). ¹³C-NMR (75 MHz, DMSO-d6) δ: 178.70, 174.73, 168.28, 147.89, 144.93, 134.34, 128.55, 127.56, 121.15, 118.99, 108.32, 104.90, 69.98, 68.16, 44.59, 37.95, 36.74, 9.50. MS: m/z (ES): 395.05 [MH]⁺.

### Intermediate 4

### 7-Chloro-1-cyclopropyl-6-(2-hydroxy-ethoxy)-4-oxo-1,4-dihydro-quinoline-3-carboxylic acid (A) and 1-Cyclopropyl-6-fluoro-7-(2-hydroxy-ethoxy)-4-oxo-1,4-dihydro-quinoline-3-carboxylic acid (B)

To a mixture of DMSO (5 mL) and ethyleneglycol (6 ml), KO^{t}Bu (1.6 g, 14.23 mmol) was added portionwise over 10 min, and then heated to 90°C. To the mixture, 7-chloro-1-cyclopropyl-6-fluoro-4-oxo-1,4-dihydro-quinoline-3-carboxylic acid (1.0 g) was added portionwise over 20 min, the temperature was increased to 105 °C and the mixture was stirred for 6 h. Water (30 mL) was added to the reaction solution and the pH of the solution was adjusted to pH=5. The resulting solution was left in the refrigerator overnight. The precipitate obtained was filtered, washed with cold water, and dried affording a 2:1 mixture of **Intermediate 4A** and **Intermediate 4B** (1.0 g).

Part of the crude product (700 mg) was dissolved in EtOH (15 mL) by heating to the reflux. The resulting solution was cooled to 30°C and a first precipitation occurred. The precipitate was filtered, washed with cold EtOH and dried under reduced pressure. **Intermediate 4A** (204 mg) was obtained as a white solid.

¹H-NMR (500 MHz, DMSO-d6) δ: 15.06 (s, 1H), 8.71 (s, 1H), 8.40 (s, 1H), 7.86 (s, 1H), 4.97 (t, 1H), 4.25 (t, 2H), 3.87 (m, 1H), 3.82 (q, 2H), 1.32 (m, 2H), 1.20 (m, 2H). ¹³C-NMR (75 MHz, DMSO-d6) δ: 176.61, 165.67, 152.47, 147.54, 135.34, 129.48, 124.95, 120.02, 106.90, 106.66, 71.22, 59.15, 35.99, 7.46. MS; m/z (ES): [MH]⁺

### Intermediate 5: 7-Chloro-6-[2-(2-cyano-ethoxy)-ethoxy]-1-cyclopropyl-4-oxo-1,4-dihydro-quinoline-3-carboxylic acid

To a suspension of **Intermediate 4A** (2 g) in acrylonitrile (40 mL) was added DBU (2.3 ml). The reaction mixture was stirred at 80°C for 24 h. The acrylonitrile was evaporated under reduced pressure. Isopropanol (30 mL) was added to the residue and the pH of the solution was adjusted to pH=5 by adding 2M HCl, during which the product precipitated. The precipitate was filtered, washed with water, and dried affording **Intermediate 5** (1.7 g) as a white solid.

¹H-NMR (500 MHz, DMSO-d6) δ: 8.68 (s, 1H), 8.38 (s, 1 H), 7.84 (s, 1H), 4.38 (t, 2H), 3.91 (t, 2H), 3.86 (m, 1H), 3.75 (t, 2H), 2.79 (t, 2H), 1.32 (m, 2H), 1.20 (m, 2H). ¹³C-NMR (75 MHz, DMSO-d6) δ: 176.63, 165.65, 152.18, 147.61, 135.50, 129.44, 124.97, 120.04, 119.11, 106.96, 106.80, 69.02, 68.30, 65.49, 35.99, 18.06, 7.46. MS; m/z (ES): 377.03 [MH]⁺

### Intermediate 6: 6-[2-(2-Carboxy-ethoxy)-ethoxy]-7-chloro-1-cyclopropyl-4-oxo-1,4-dihydro-quinoline-3-carboxylic acid

A solution of **Intermediate 5** (1.10 g) in a mixture of conc. H₂SO₄ (10 mL) and H₂O (20 mL) was stirred at 75 °C for 24 h. The pH of the reaction mixture was adjusted to 0.2 with 40% NaOH, during which the product precipitated. The precipitate was filtered, washed with water, and dried affording **Intermediate 6** (0.8 g) as a white solid.

¹H-NMR (300 MHz, DMSO-d6) δ: 15.0 (s, 1H), 11.8 (s, 1H), 8.69 (s, 1H), 8.38 (s, 1H), 7.85 (s, 1H), 4.35 (m, 2H), 3.91-3.82 (m, 3H), 3.74 (dt, 2H), 2.49 (m, 2H), 1.31 (m, 2H), 1.19 (m, 2H). MS; m/z (ES): 396.02 [MH]⁺.

### Intermediate 7: 4"-Hydroxyimino-9a-azithromycin

4"-Keto-9a-azithromycin (5.2 g, 0.007 mol) was treated with hydroxylamine hydrochloride (2.4 g) in MeOH (260 mL) for 3.5 hours at room temperature. The methanol was evaporated and the residue dissolved in EtOAc (200 mL). Water was then added (200 mL) and extracted at pH 9.8. Solvent was removed affording crude product (5.39 g). After purification by flash chromatography (DCM-MeOH-NH₄OH = 90:9: 0.5) the title compound (2.4 g) was obtained; MS (ES+) m/z:[MH]⁺ = 762.33.

### Intermediates 8 and 9: 4"-(S) and 4"-(R)-Amino-9a-azithromycin

**Intermediate 7** (2.0 g, 0.0026 mol) was dissolved in acetic acid (100 mL) and hydrogenated over 2.0 g platinum oxide at 1150 psi. for 48 hours at room temperature. This was followed by a fresh addition of 0.8 g of platinum oxide and the reaction was continued for another 24 hours under 1150 psi. Since TLC shown some starting compound a further 0.8 g platinum oxide was added and reduction continued for a further 24 hours at the same pressure. The reaction mixture was filtered and acetic acid was removed under vacuum. The residue was dissolved in 100 mL of CHCL₃ and 50 mL of water and extracted at pH 5 and 10. Evaporation of extract at pH 10 afforded a mixture of 4"-(S) and 4"-(R) amines (1.96 g).

After purification by column chromatography (DCM-MeOH-NH₄OH = 90:9: 1.5) two separate isomers were isolated: **Intermediate 8** with Rf=0.67, δ 4.10, dq, H-5", 4"-(S)-amine and **Intermediate 9** with Rf=0.63, δ 4.57, dq, H-5", 4"-(*R*)-amine.
MS (ES+) m/z : [MH]⁺ = 748.36.

### Intermediate 10: 4"-(S)-Amino-9a-azithromycin 11,12-cyclic carbonate

**Intermediate 8** (0.1 g, 0.13 mmol) was dissolved in benzene (4 mL) and then ethylene carbonate (0.09 g) and K₂CO₃ (0.11g) were added to the reaction mixture. The reaction mixture was heating at 80°C overnight. After filtration, the filtrate was rinsed twice with H₂O and then evaporated giving 0.097 mg of the title product. MS m/z = 774.4 (MH⁺).

### Intermediate 11: 4"-(R)-Amino-9a-azithromycin 11,12-cyclic carbonate

Starting from **Intermediate 9** (0.080 g), the title compound was prepared according the procedure described for **Intermediate 10.** MS m/z = 774.4 (MH+).

### Intermediate 12 and Intermediate 13: 6-[(2-Amino-ethyl)amino]-7-chloro-1-cyclopropyl-1,4-dihydro-4-oxo-quinoline-3-carboxylic acid hydrochloride (12) and 7-[(2-amino-ethyl)amino]-1-cyclopropyl-1,4-dihydro-6-fluoro-4-oxo-quinoline-3-carboxylic acid hydrochloride (13)

7-Chloro-1-cyclopropyl-1,4-dihydro-6-fluoro-4-oxo-quinoline-3-carboxylic acid (56.3 g) and ethylenediamine (36 g) were dissolved in N,N-dimethylacetamide (650 mL) at 100°C and stirred for 8.5 h at 115°C. Water (700 mL) was added to the reaction mixture cooled at room temperature. The reaction mixture was stirred at room temperature for 2 h, cooled at 0-5°C and stirred for 1 h. The precipitate obtained was filtered, washed with cold water, cold EtOH, and dried at 110°C under reduced pressure for 1 h. The crude product was treated with HCl (6% aqueous solution) heating for 1 h in the presence of charcoal. After filtration, the solution was cooled to 35-40°C and a first precipitation occurred. The precipitate was filtered, washed with water and dried at 110°C for 1 h. **Intermediate 12** (6.4 g) was obtained as a hydrochloride salt. The mother liquors, after first precipitation, were cooled at room temperature and stirred overnight. The precipitate was filtered, washed with water and dried at 110°C for 1 h to give a mixture containing **Intermediates 12 and 13** (14.18 g). **Intermediate 12:** ¹H-NMR (300 MHz, CF₃COOD) d: 8.94 (s, 1H), 8.40 (s, 1H), 7.40 (s, 1H), 3.85 (m, 1H), 3.76 (m, 2H), 5.45 (m, 2H), 1.42 (m, 2H), 1.77 (m, 2H).

### Intermediate 14: 6-{2-[(2-Carboxy-ethyl)-methyl-amino]-ethylamino}-7-chloro-1-cyclopropyl-4-oxo-1,4-dihydro-quinoline-3-carboxylic acid methyl ester

### a) 6-(2-Amino-ethylamino)-7-chloro-1-cyclopropyl-4-oxo-1,4-dihydro-quinoline-3-carboxylic acid methyl ester.

A suspension of **Intermediate 12** (120 mg) in a solution of HCl in MeOH (3%, 30 mL) was sonicated in an ultrasonic water bath at 60°C for 3 h and then at room temperature for 48 h. The solvent was evaporated under reduced pressure and the crude product was purified by flash chromatography (eluent: MeOH/DCM/NH₄OH 9/5/0.5) affording the title compound (80 mg). ¹H-NMR (300 MHz, DMSO-d6) d: 8.37 (s, 1 H), 8.04 (s, 1H), 7.36 (s, 1H), 5,77 (t, 1H), 3,37 (s, 3H, O-Me), 3.64 (m, 1H), 3,20 (q, 2H), 2,85 (t, 2H), 1.23 (m, 2H), 1,08 (m, 2H).

### b) 6-[2-(2-Carboxy-ethylamino)-ethylamino]-7-chloro-1-cyclopropyl-4-oxo-1,4-dihydro-quinoline-3-carboxylic acid methyl ester.

To a solution of acrylic acid (0.5 ml, 7.44 mmol) in 2-propanol (120 ml) was added Et₃N (2 ml), H₂O (20 ml) and **Intermediate 14a** (2.5 g), and the mixture was heated at 60°C for 24 h. Et₃N (3ml) was added to the reaction mixture and the mixture was heated at 60°C for an additional 24 h. The solvents were concentrated under reduced pressure, H₂O (70 ml) was added to the residue, the pH was adjusted with 2 M NaOH to 9.5, and the mixture was extracted with EtOAc (2 x 30 ml). The EtOAc was discharged, the pH of the aqueous solution was adjusted with 2 M HCI to 3 and the solution was extracted with EtOAc (2 x 30 ml). The product was in the aqueous solution. Thus, the aqueous solution was concentrated under reduced pressure, methanol was added to the residue and the mixture was stirred for 15 min, filtered and the filtrate was concentrated under reduced pressure, affording the crude product. Part of the crude product (1.0 g) was purified on an SPE-column to afford the title compound.

### c) 6-{2-[(2-Carboxy-ethyl)-methyl-amino]-ethylamino}-7-chloro-1-cyclopropyl-4-oxo-1,4-dihydro-quinoline-3-carboxylic acid methyl ester.

To the rest of the crude **Intermediate 14b** were added acetone (60 mL), formaldehyde (0.60 mL, 36 % solution) and formic acid (0.60 mL). This mixture was heated at 55°C for 24 hours. The aqueous solution was concentrated under reduced pressure, methanol was added to the residue and the mixture was stirred for 15 min, filtered and the filtrate was concentrated under reduced pressure. The residue was purified on an SPE-column to afford the title compound (0.84 g ). ¹³C NMR (75 MHz, DMSO) d ppm: 174.3, 172.0, 165.1. 146.6, 141.9, 131.4, 128.0, 124.6. 118.1, 107.8, 104.5. 57.7, 54.4, 53.1. 51.1, 41.3, 34.6, 33.2, 7.3.

### Example 1: 4"-(S)-{3-[2-(3-Carboxy-7-chloro-1-cyclopropyl-4-oxo-1,4-dihydroquinolin-6-ylamino)-ethoxy]-propionylamino}-4"-deoxyazithromycin

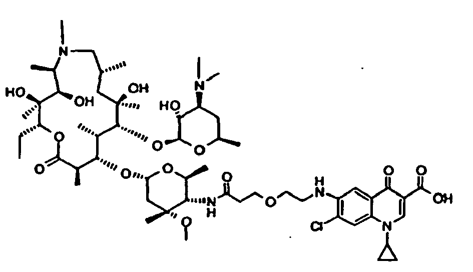

To a DMF (3 mL) solution of **Intermediate 3** (0.106 g, 0.268 mmol), DCC (0.110 g, 0.53 mmol), **Intermediate 8** (0.100 g, 0.134 mmol) and DMAP (10 mg) were added and the reaction mixture was stirred for 20 hours at room temperature. Water and EtOAc were added and the layers were separated. The water layer was extracted with EtOAc and the combined organic layers were dried over K₂CO₃ and then evaporated. The residue was precipitated from EtOAc/n-hexane yielding 80 mg of crude product which was purified by column chromatography (SPE-column, gradient polarity: 100 % DCM to DCM:MeOH:NH₃ = 90:9:0.5) yielding 50 mg of product which was precipitated from EtOAc:n-hexane yielding 30 mg of pure **Example 1**; MS; m/z (ES): 1124.20 [MH]⁺.

### Example 2: 4"-(R)-{3-[2-(3-Carboxy-7-chloro-1-cyclopropyl-4-oxo-1,4-dihydroquinolin-6-ylamino)-ethoxy]-propionylamino}-4"-deoxyazithromycin

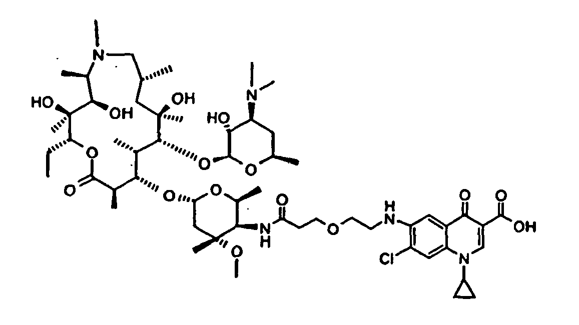

To a DMF (3 mL) solution of **Intermediate 3** (0.106 g, 0.268 mmol), DCC (0.110 g, 0.53 mmol), **Intermediate 9** (0.100 g, 0.134 mmol) and DMAP (10 mg) were added and the reaction mixture was stirred for 20 hours at room temperature. Water and EtOAc were added and the layers were separated. The water layer was extracted with EtOAc and the combined organic layers were dried over K₂CO₃ and then evaporated. The residue was precipitated from EtOAc/n-hexane yielding 80 mg of crude product which was purified by column chromatography (SPE-column, gradient polarity: 100 % DCM to DCM:MeOH:NH₃ = 90:9:0.5) yielding of pure **Example 2** (60 mg); MS; m/z (ES): 1124.30 [MH]⁺.

### Example 3: 4"-(S)-{3-[2-(3-Carboxy-7-chloro-1 -cyclopropyl-4-oxo-1,4-dihydroquinolin-6-yloxy)-ethoxy]-propionylamino}-4"-deoxyazithromycin

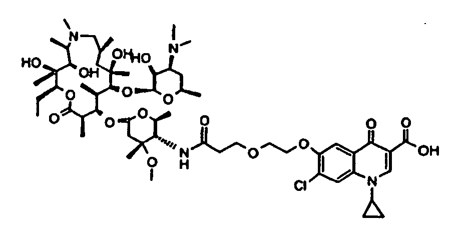

To a solution of **Intermediate 8** (75 mg, 0.01 mmol) in DCM (3 mL) was added 1,3-dicyclohexylcarbodiimide (0.082 g, 0.082 mmol). **Intermediate 6** was added (64 mg) followed by DMAP (10 mg). The reaction mixture was stirred at room temperature for 24 h. H₂O (25 mL) was added to the reaction mixture. The aqueous phase was washed with DCM (2x30 mL). The combined organic layers were concentrated under reduced pressure and the residue was purified on silica gel using DCM/MeOH/NH₄OH 90/10/0.5 affording **Example 3** (37 mg) as a white solid; MS; m/z (ES):1125.46 [MH]⁺.

### Example 4: 4"-(R)-{3-[2-(3-Carboxy-7-chloro-1-cyclopropyl-4-oxo-1,4-dihydroquinolin-6-yloxy)-ethoxy]-propionylamino}-4"-deoxyazithromycin

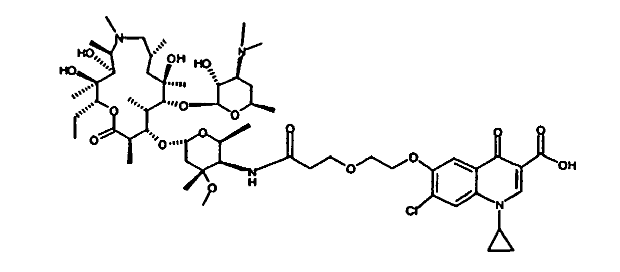

To a solution of **Intermediate 9** ( 0.20 g) in DCM (6 mL), **Intermediate 6** (0.141 g), DMAP (0.013 g) and DCC (0.111 g) were added and the reaction mixture was stirred at room temperature for 48 hours. The solvent was evaporated yielding 0.480 g of crude product.

After purification by column chromatography (DCM-MeOH-NH₄OH = 90:9: 1.5) the title compound was isolated.

### Example 5: 4"-(R)-{3-[2-(3-Carboxy-1-cyclopropyl-4-oxo-1,4-dihydro-quinolin-6-yloxy)-ethoxy]-propionylamino}-4"-deoxyazithromycin

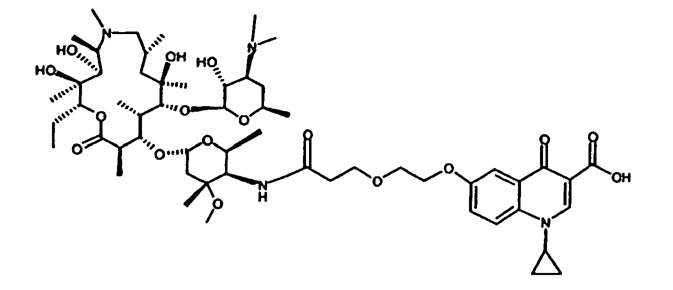

**Example 4** (80 mg) was dissolved in methanol (11 mL) and 10 % Pd/C (55 mg) was added. Hydrogenolysis was performed at 4 x 10⁵ Pa for 4 h. The reaction mixture was filtered and the filtrate evaporated yielded 0.09 g of the title product. MS m/z = 1091.6 (MH)⁺.

### Example 6: 4"-(R)-(3-{2-(3-Carboxy-1-cyclopropyl-4-oxo-1,4-dihydro-quinolin-6-yloxy)-ethoxy]-propionylamino}-4"-deoxyazithromycin 11,12-cyclic carbonate

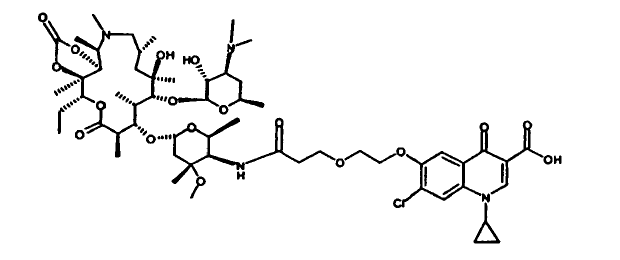

**Intermediate 11** (0.073 g, 0.094 mmol) was dissolved in DMS (2.6 mL). To the reaction solution, DCC (0.052 g), DMAP (0.0065 g) and **Intermediate 6** (0.0.065 g) were added and the reaction mixture was stirred at room temperature overnight. Filtration and evaporation of solvent yielded a crude product. The crude product was dissolved in EtOAc, H₂O was added and extracted 3xEtOAc at pH 9.3. The combined organic layers were evaporated under reduced pressure to a solid (0.099 g). Purification by column chromatography (DCM-MeOH-NH₃ = 90:9:1.5) yielded 0.047 g of the title product. (M + 2H)²⁺ m/z = 577.51.

### Example 7: 4"-(S)-{3-[2-(3-Carboxy-1-cyclopropyl-4-oxo-1,4-dihydro-quinolin-6-yloxy)-ethoxyl-propionylamino}-4"-deoxyazithromycin 11,12-cyclic carbonate

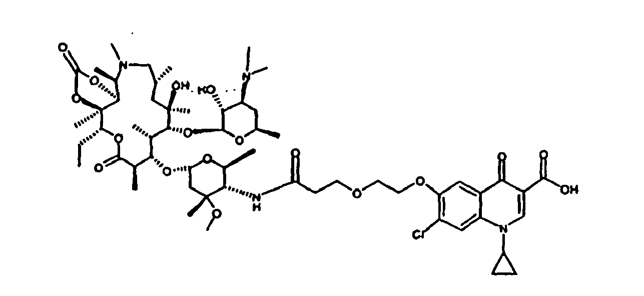

**Intermediate 10** (0.097 g, 0.12 mmol) was dissolved in DMS (4 mL). To the reaction solution, DCC (0.069 g), DMAP (0.0086 g) and **Intermediate 6** (0.0.087 g) were added and the reaction mixture was stirred at room temperature overnight. Filtration and evaporation of solvent yielded a crude product. The crude product was dissolved in EtOAc, H₂O was added and extracted 3xEtOAc at pH 9.05. The combined organic layers were evaporated under reduced pressure to a solid (0.114 g). Purification by column chromatography (DCM-MeOH-NH₃ = 90:9:1.5) yielded 0.041 g of the title product. (M + 2H)²⁺ m/z = 577.52.

### Example 8: 4"-(3-{[2-(7-Chloro-1-cyclopropyl-3-methoxycarbonyl-4-oxo-1,4-dihydroquinolin-6-ylamino)-ethyl]-methyl-amino}-propionylaminol)-4"-deoxyazithromycin

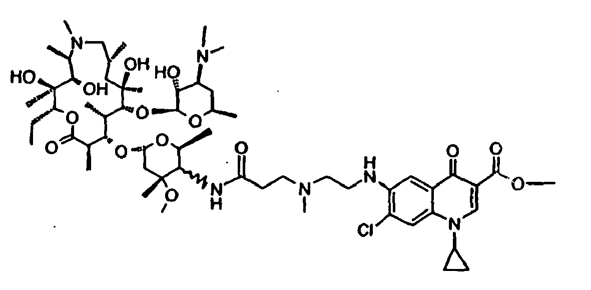

To a solution of 4"-amino-4"-deoxoazithromycin (0.11 mg, 0.26 mmol, mixture of **Intermediates 8** and **9**) in DCM (2 mL) was added 1,3-dicyclohexylcarbodiimide (0.108 g, 0.052 mmol). **Intermediate 14c** (80 mg) was then added, followed by 4-dimethylamino pyridine (10 mg). The reaction mixture was stirred at room temperature for 24 hours. H₂O (25 mL) was added to the reaction mixture and the aqueous phase was washed with DCM (2x30 mL). The combined organic layers were concentrated under reduced pressure and the residue was purified on silica gel using: DCM/MeOH/NH₄OH 90/10/0.5 affording the title compound (12 mg). TLC (DCM-MeOH-NH₄OH = 90:9: 1.5): R_{f} = 0.616. MS m/z = 1152.2 (MH)⁺.

### Biological Data

Using a standard broth dilution method in microtitre, compounds were tested for antibacterial activity. The compounds in the above examples gave minimum inhibitory concentrations (MICs) less than 1 microgram per millilitre against erythromycin-sensitive and erythromycin-resistant strains of *Streptococcus pneumoniae* and *Streptococcus pyogenes.*

In addition, the MIC (µg/mL) of test compounds against various organisms was determined including:
*S. aureus* Smith ATCC 13709, *S. pneumoniae* SP030, *S. pyogenes* 3565, *E. faecalis* ATCC 29212, *H. influenzae* ATCC 49247, *M. catarrhalis* ATCC 23246.

Examples 1 to 3 have an MIC ≤1 *µ*g/mL against *S.* aureus Smith ATCC 13709, *S. pneumoniae* SP030, *S. pyogenes* 3565 and *E. faecalis* ATCC 29212.

Examples 1 to 3 have an MIC ≤2 *µ*g/mL against *H.* influenzae ATCC 49247 and *M. catarrhalis* ATCC 23246.

## Claims

1. A compound of formula (I) 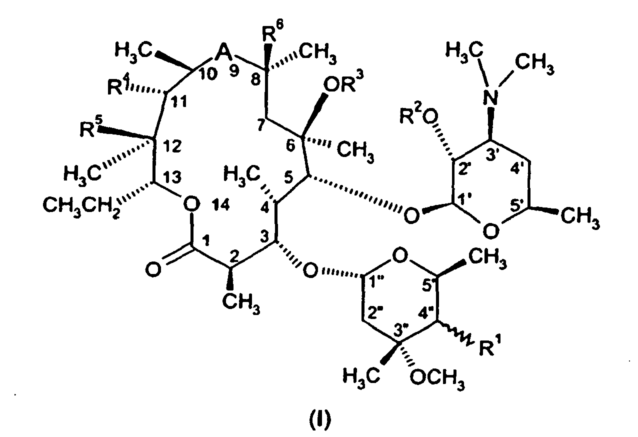 wherein
A is a bivalent radical selected from -C(O)NH-, -NHC(O)-, -N(R⁷)-CH₂- and -CH₂-N(R⁷)-;
R¹ is -NHC(O)(CH₂)_{d}XR⁸;
R² is hydrogen;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R⁹,
R⁵ is hydroxy, or
R⁴ and R⁵ taken together with the intervening atoms form a cyclic group having the following structure: 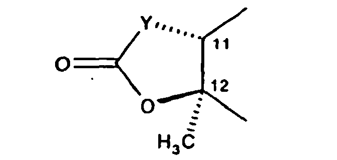 wherein Y is a bivalent radical selected from -CH₂-, -CH(CN)-, -O-, -N(R¹⁰)- and - CH(SR¹⁰)-, with proviso that when A is -NHC(O)-, -N(R⁷)-CH₂- or -CH₂-N(R⁷)-, Y is -O-;
R⁶ is hydrogen or fluorine;
R⁷ is hydrogen or C₁₋₆alkyl;
R⁸ is a heterocyclic group having the following structure: 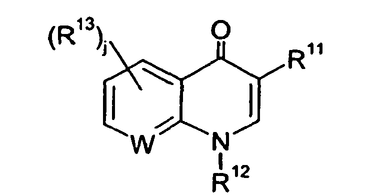 or 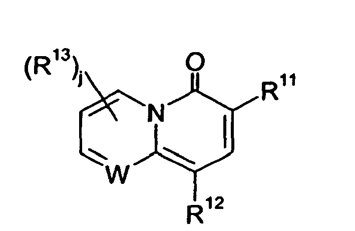
R⁹ is hydrogen or C₁₋₆alkyl;
R¹⁰ is hydrogen or C₁₋₄alkyl optionally substituted by a group selected from optionally substituted phenyl, optionally substituted 5 or 6 membered heteroaryl and optionally substituted 9 to 10 membered fused bicyclic heteroaryl;
R¹¹ is hydrogen, -C(O)OR¹⁴, -C(O)NHR¹⁴, -C(O)CH₂NO₂ or -C(O)CH₂SO₂R⁷;
R¹² is hydrogen, C₁₋₄alkyl optionally substituted by hydroxy or C₁₋₄alkoxy, C₃₋₇cycloalkyl, or optionally substituted phenyl or benzyl;
R¹³ is halogen, C₁₋₄alkyl, C₁₋₄thioalkyl, C₁₋₄alkoxy, -NH₂, -NH(C₁₋₄alkyl) or -N(C₁₋₄alkyl)₂;
R¹⁴ is hydrogen,
C₁₋₆alkyl optionally substituted by up to three groups independently selected from halogen, cyano, C₁₋₄alkoxy optionally substituted by phenyl or C₁₋₄alkoxy, - C(O)C₁₋₆alkyl, -C(O)OC₁₋₆alkyl, -OC(O)C₁₋₆alkyl, -OC(O)OC₁₋₆alkyl, - C(O)NR¹⁷R¹⁸, -NR¹⁷R¹⁸ and phenyl optionally substituted by nitro or -C(O)OC₁₋₆alkyl,
-(CH₂)_{w}C₃₋₇cycloalkyl,
-(CH₂)_{w}heterocyclyl,
-(CH₂)_{w}heteroaryl,
-(CH₂)_{w}aryl,
C₃₋₆alkenyl, or
C₃₋₆alkynyl;
R¹⁵ is hydrogen, C₁₋₄alkyl, C₃₋₇cycloalkyl, optionally substituted phenyl or benzyl, acetyl or benzoyl;
R¹⁶ is hydrogen or R¹³, or R¹⁶ and R¹² are linked to form the bivalent radical -O(CH₂)₂-or -(CH₂)ₜ-;
R¹⁷ and R¹⁸ are each independently hydrogen or C₁₋₆alkyl optionally substituted by phenyl or -C(O)OC₁₋₆alkyl, or
R¹⁷ and R¹⁸, together with the nitrogen atom to which they are bound, form a 5 or 6 membered heterocyclic group optionally containing one additional heteroatom selected from oxygen, nitrogen and sulfur;
X is -U(CH₂)ᵥB-, -U(CH₂)ᵥ- or a group selected from: 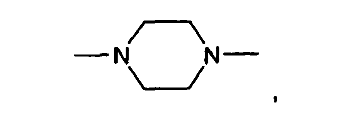 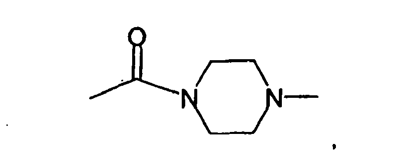 and 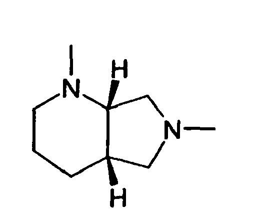
U and B are independently a divalent radical selected from -N(R¹⁵)-, -O-, -S(O)_{z}-, - N(R¹⁵)C(O)-, -C(O)N(R¹⁵)- and -N[C(O)R¹⁵]-;
W is -C(R¹⁶)- or a nitrogen atom;
d is 0 or an integer from 1 to 5;
e is an integer from 2 to 4;
j and z are each independently integers from 0 to 2;
w is an integer from 0 to 4;
t is 2 or 3;
v is an integer from 1 to 8;
or a pharmaceutically acceptable derivative thereof.

2. A compound according to claim 1 wherein A is -N(R⁷)-CH₂-.

3. A compound according to claim 1 or claim 2 wherein X is -O(CH₂)₂NH- or - O(CH₂)₂O-.

4. A compound according to any one of the preceding claims wherein d is 2.

5. A compound according to any one of the preceding claims wherein R⁸ is a heterocyclic group of the following formula: 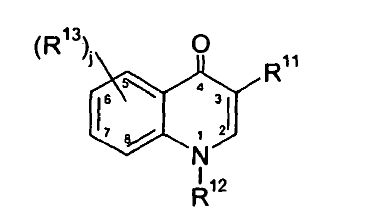 wherein the heterocyclic is linked in the 6 or 7 position and j, R¹¹, R¹² and R¹³ are as defined in claim 1.

6. A compound according to claim 1 as defined in any one of Examples 1 to 8, or a pharmaceutically acceptable derivative thereof.

7. A compound selected from:
4"-(S)-{3-[2-(3-carboxy-7-chloro-1-cyclopropyl-4-oxo-1,4-dihydro-quinolin-6-ylamino)-ethoxy]-propionylamino}-4"-deoxyazithromycin;
4"-(R)-(3-[2-(3-carboxy-7-chloro-1-cyclopropyl-4-oxo-1,4-dihydro-quinolin-6-ylamino)-ethoxy]-propionylamino)-4"-deoxyazithromycin;
4"-(S)-{3-[2-(3-carboxy-7-chloro-1-cyclopropyl-4-oxo-1,4-dihydro-quinolin-6-yloxy)-ethoxy]propionylamino}-4"-deoxyazithromycin;
4"-(S)-{3-[2-(3-carboxy-1-cyclopropyl-4-oxo-1,4-dihydro-quinolin-6-yloxy)-ethoxy]-propionylamino}-4"-deoxyazithromycin 11,12-cyclic carbonate; and
4"-(3-{[2-(7-chloro-1-cyclopropyl-3-methoxycarbonyl-4-oxo-1,4-dihydro-quinolin-6-ylamino)-ethyl]-methyl-amino}-propionylamino)-4"-deoxyazithromycin;
or a pharmaceutically acceptable derivative thereof.

8. A compound selected from:
4"-(*S*)-{3-[2-3-Carboxy-7-chloro-1-cyclopropyl-4-oxo-1,4-dihydro-quinolin-6-ylamino)-ethoxy]-propionylamino}-4"-deoxyazithromycin
4"-(R)-{3-[2-(3-Carboxy-7-chloro-1-cyclopropyl-4-oxo-1,4-dihydro-quinolin-6-ylamino)-ethoxy]-propionylamino}-4"-deoxyazithromycin
4"-(S)-{3-[2-(3-Carboxy-7-chloro-1-cyclopropyl-4-oxo-1,4-dihydro-quinolin-6-yloxy)-ethoxy]-propionylamino}-4"-deoxyazithromycin
4"-(*R*)-{3-[2-(3-Carboxy-7-chloro-1-cyclopropyl-4-oxo-1,4-dihydro-quinolin-6-yloxy)-ethoxy]-propionylamino}-4"-deoxyazithromycin
4"-(*R*)-{3-[2-(3-Carboxy-1-cyclopropyl-4-oxo-1,4-dihydro-quinolin-6-yloxy)-ethoxy]-propionylamino}-4"-deoxyazithromycin
4"-(*R*)-{3-[2-(3-Carboxy-1-cyclopropyl-4-oxo-1,4-dihydro-quinolin-6-yloxy)-ethoxy]-propionylamino}-4"-deoxyazithromycin 11,12-cyclic carbonate
4"-(*S*)-{3-[2-(3-Carboxy-1-cyclopropyl-4-oxo-1,4-dihydro-quinolin-6-yloxy)-ethoxy]-propionylamino}-4"-deoxyazithromycin 11,12-cyclic carbonate
4"-(3-{[2-(7-Chloro-1-cyclopropyl-3-methoxycarbonyl-4-oxo-1,4-dihydro-quinolin-6-ylamino)-ethyl]-methyl-amino}-propionylamino)-4"-deoxyazithromycin
or a pharmaceutically acceptable derivative thereof.

9. A process for the preparation of a compound as claimed in claim 1 which comprises:
a) reacting a compound of formula (II) 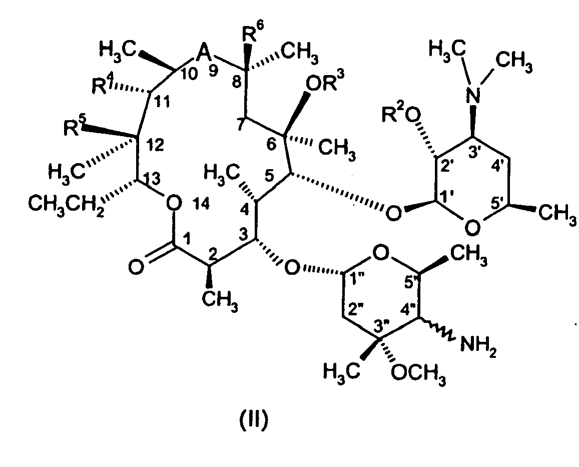
HOC(O)(CH₂)_{d}X^{a}R^{8a} (III)
with a suitable activated derivative of the acid (III), wherein X^{a} and R^{8a} are X and R⁸ as defined in claim 1 or groups convertible to X and R⁸ to produce a compound of formula (I) wherein d is an integer from 1 to 5;
b) reacting a compound of formula (II) with a suitable activated derivative of the carboxylic acid HOC(O)N(R¹⁵)(CH₂)ᵥB^{a}R^{8a} (IV) to produce a compound of formula (I) wherein d is 0 and U is -C(O)N(R¹⁵)-;
c) reacting a compound of formula (II) with an isocyanate OCN(CH₂)ᵥB^{a}R^{8a} to produce a compound of formula (I) wherein d is 0 and U is -NH-;
d) reacting a compound of formula (II) with a carbamoyl chloride CIC(O)N(R¹⁵)(CH₂)ᵥB^{a}R^{8a} to produce a compound of formula (I) wherein d is 0 and U is -N(R¹⁵)-;
e) reacting a compound of formula (II) with a chloroformate CIOC(O)O(CH₂)ᵥB^{a}R^{8a} to produce a compound of formula (I) wherein d is 0 and U is -O-;
f) reacting a compound of formula (V) 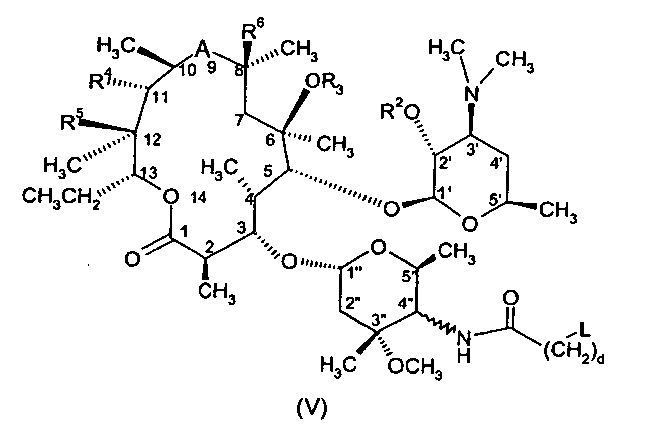 with a compound of formula X^{a}R^{8a} (VI), wherein R^{8a} is R⁸ as defined in claim 1 or a group convertible to R⁸ and X^{a} is -U(CH₂)ᵥ- or -U(CH₂)ᵥB-, or a group convertible to - U(CH₂)ᵥ- or -U(CH₂)ᵥB-, in which U is a group selected from -N(R¹⁵)- and -S-, and L is suitable leaving group, to produce a compound of formula (I) wherein U is a group selected from -N(R¹⁵)- and -S-; or
g) converting one compound of formula (I) into another compound of formula (I),
and thereafter, if required, subjecting the resulting compound to one or more of the following operations:
i) removal of the protecting group R²,
ii) conversion of X^{a}R^{8a} to XR⁸,
iii) conversion of B^{a}R^{8a} to BR⁸, and
iv) conversion of the resultant compound of formula (I) into a pharmaceutically acceptable derivative thereof.

10. A compound as claimed in any one of claims 1 to 8 for use in therapy.

11. The use of a compound as claimed in any one of claims 1 to 8 in the manufacture of a medicament for use in the treatment or prophylaxis of systemic or topical microbial infections in a human or animal body.

12. A pharmaceutical composition comprising at least one compound as claimed in any one of claims 1 to 8 in association with a pharmaceutically acceptable excipient, diluent and/or carrier.

13. A compound of formula (IA) 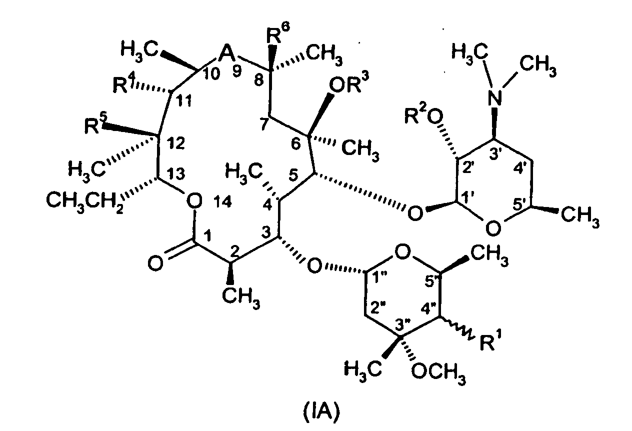 wherein
A is a bivalent radical selected from -C(O)NH-, -NHC(O)-, -N(R⁷)-CH₂- and -CH₂-N(R⁷)-;
R¹ is -NHC(O)(CH₂)_{d}XR⁸;
R² is hydrogen;
R³ is hydrogen, C₁₋₄alkyl, or C₃₋₆alkenyl optionally substituted by 9 to 10 membered fused bicyclic heteroaryl;
R⁴ is hydroxy, C₃₋₆alkenyloxy optionally substituted by 9 to 10 membered fused bicyclic heteroaryl, or C₁₋₆alkoxy optionally substituted by C₁₋₆alkoxy or -O(CH₂)ₑNR⁷R⁹,
R⁵ is hydroxy, or
R⁴ and R⁵ taken together with the intervening atoms form a cyclic group having the following structure: 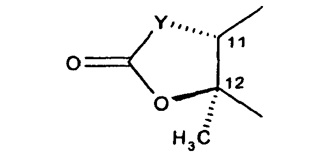 wherein Y is a bivalent radical selected from -CH₂-, -CH(CN)-, -O-, -N(R¹⁰)- and - CH(SR¹⁰)-, with proviso that when A is -NHC(O)-, -N(R⁷)-CH₂- or -CH₂-N(R⁷)-, Y is -O-;
R⁶ is hydrogen or fluorine;
R⁷ is hydrogen or C₁₋₆alkyl;
R⁸ is a heterocyclic group having the following structure: 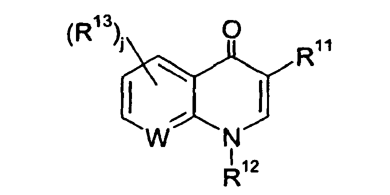 or 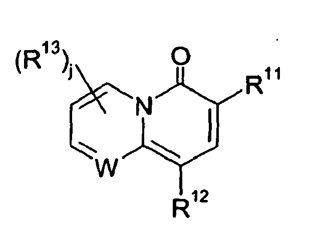
R⁹ is hydrogen or C₁₋₆alkyl;
R¹⁰ is hydrogen or C₁₋₄alkyl substituted by a group selected from optionally substituted phenyl, optionally substituted 5 or 6 membered heteroaryl and optionally substituted 9 to 10 membered fused bicyclic heteroaryl;
R¹¹ is hydrogen, -C(O)OR¹⁴, -C(O)NHR¹⁴ or -C(O)CH₂NO₂;
R¹² is hydrogen, C₁₋₄alkyl optionally substituted by hydroxy or C₁₋₄alkoxy, C₃₋₇cycloalkyl, or optionally substituted phenyl or benzyl;
R¹³ is halogen, C₁₋₄alkyl, C₁₋₄thioalkyl, C₁₋₄alkoxy, -NH₂, -NH(C₁₋₄alkyl) or -N(C₁₋₄alkyl)₂;
R¹⁴ is hydrogen or C₁₋₆alkyl optionally substituted by up to three groups independently selected from halogen, C₁₋₄alkoxy, -OC(O)C₁₋₆galkyl and -OC(O)OC₁₋₆alkyl;
R¹⁵ is hydrogen, C₁₋₄alkyl, C₃₋₇cycloalkyl, optionally substituted phenyl or benzyl, acetyl or benzoyl;
R¹⁶ is hydrogen or R¹³, or R¹⁶ and R¹² are linked to form the bivalent radical -O(CH₂)₂-or -(CH²)ₜ-;
X is -U(CH₂)ᵥB-, -U(CH₂)ᵥ- or a group selected from: 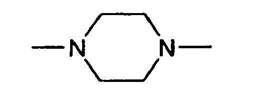 and 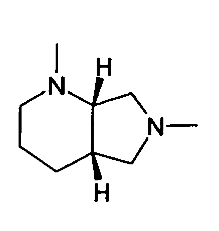
U and B are independently a divalent radical selected from -N(R¹⁵)-, -O-, -S(O)_{z}-, - N(R¹⁵)C(O)-, -C(O)N(R¹⁵)- and -N[C(O)R¹⁵]-;
W is -C(R¹⁶)- or a nitrogen atom;
d is 0 or an integer from 1 to 5;
e is an integer from 2 to 4;
j and z are each independently integers from 0 to 2;
t is 2 or 3;
v is an integer from 2 to 8;
or a pharmaceutically acceptable derivative thereof.

## Patentansprüche

1. Verbindung der Formel (I): 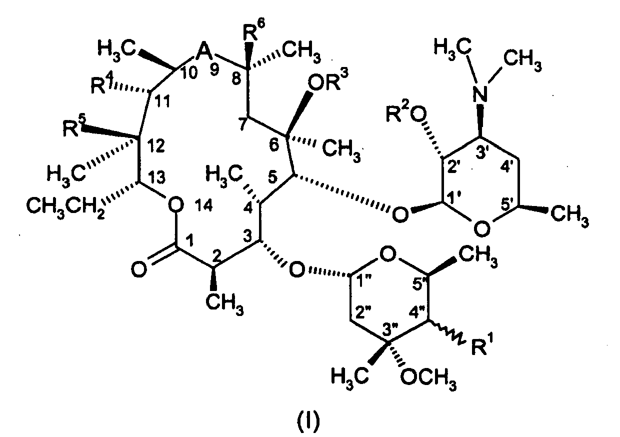 worin:
A ein zweiwertiger Rest ist, der aus -C(O)NH-, -NHC(O)-, -N(R⁷)-CH₂- und -CH₂-N(R⁷)- ausgewählt ist;
R¹ -NHC(O)(CH₂)_{d}XR⁸ ist;
R² Wasserstoff ist;
R³ Wasserstoff, C₁₋₄-Alkyl oder gegebenenfalls mit 9-bis 10-gliedrigem kondensiertem bicyclischem Heteroaryl substituiertes C₃₋₆-Alkenyl ist;
R⁴ Hydroxy, gegebenenfalls mit 9- bis 10-gliedrigem kondensiertem bicyclischem Heteroaryl substituiertes C₃₋₆-Alkenyloxy oder gegebenenfalls mit C₁₋₆-Alkoxy oder -O(CH₂)ₑNR⁷R⁹ substituiertes C₁₋₆-Alkoxy ist;
R⁵ Hydroxy ist oder
R⁴ und R⁵ zusammen mit den dazwischenliegenden Atomen eine cyclische Gruppe mit der folgenden Struktur bilden: 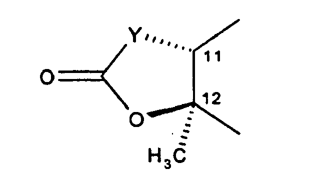 worin Y ein zweiwertiger Rest ist, der aus -CH₂-, -CH(CN)-, -O-, -N(R¹⁰)- und -CH(SR¹⁰)- ausgewählt ist, mit der Maßgabe, daß dann, wenn A -NHC(O)-, -N(R⁷)-CH₂- oder -CH₂-N(R⁷)- ist, Y -O- ist;
R⁶ Wasserstoff oder Fluor ist;
R⁷ Wasserstoff oder C₁₋₆-Alkyl ist;
R⁸ eine heterocyclische Gruppe mit der folgenden Struktur ist: 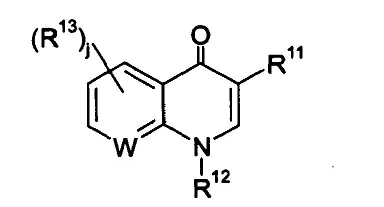 oder 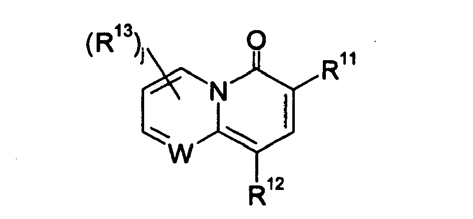
R⁹ Wasserstoff oder C₁₋₆-Alkyl ist;
R¹⁰ Wasserstoff oder C₁₋₄-Alkyl ist, das gegebenenfalls mit einer Gruppe substituiert ist, die aus gegebenenfalls substituiertem Phenyl, gegebenenfalls substituiertem 5- oder 6-gliedrigem Heteroaryl und gegebenenfalls substituiertem 9- bis 10-gliedrigem kondensiertem bicyclischem Heteroaryl ausgewählt ist;
R¹¹ Wasserstoff, -C(O)OR¹⁴, -C(O)NHR¹⁴, -C(O)CH₂NO₂ oder -C(O)CH₂SO₂R⁷ ist;
R¹² Wasserstoff, C₁₋₄-Alkyl, das gegebenenfalls mit Hydroxy-oder C₁₋₄-Alkoxy substituiert ist, C₃₋₇-Cycloalkyl oder gegebenenfalls substituiertes Phenyl oder Benzyl ist;
R¹³ Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂ ist;
R¹⁴ Wasserstoff,
C₁₋₆-Alkyl, das gegebenenfalls mit bis drei Gruppen substituiert ist, die unabhängig aus Halogen, Cyano, gegebenenfalls mit Phenyl oder C₁₋₄-Alkoxy substituiertem C₁₋₄-Alkoxy, -C(O)C₁₋₆-Alkyl,
-C(O)OC₁₋₆-Alkyl, -OC(O)C₁₋₆-Alkyl, -OC(O)OC₁₋₆-Alkyl, -C(O)NR¹⁷R⁸¹, -NR¹⁷R¹⁸ und gegebenenfalls mit Nitro oder -C(O)OC₁₋₆-Alkyl substituiertem Phenyl ausgewählt sind,
-(CH₂)_{w}C₃₋₇-Cycloalkyl,
-(CH₂)_{w}Heterocyclyl,
-(CH₂)_{w}Heteroaryl,
-(CH₂)_{w}Aryl,
C₃₋₆-Alkenyl oder
C₃₋₆-Alkinyl ist;
R¹⁵ Wasserstoff, C₁₋₄-Alkyl, C₃₋₇-Cycloalkyl, gegebenenfalls substituiertes Phenyl oder Benzyl, Acetyl oder Benzoyl ist;
R¹⁶ Wasserstoff oder R¹³ ist oder R¹⁶ und R¹² unter Bildung des zweiwertigen Restes -O(CH₂)₂- oder -(CH₂)ₜ- verbunden sind;
R¹⁷ und R¹⁸ jeweils unabhängig Wasserstoff oder gegebenenfalls mit Phenyl oder -C(O)OC₁₋₆-Alkyl substituiertes C₁₋₆-Alkyl sind oder
R¹⁷ und R¹⁸ zusammen mit dem Stickstoffatom, an das sie gebunden sind, eine 5- oder 6-gliedrige heterocyclische Gruppe bilden, die gegebenenfalls ein zusätzliches Heteroatom enthält, das aus Sauerstoff, Stickstoff und Schwefel ausgewählt ist;
X -U(CH₂)ᵥB-, -U(CH₂)ᵥ- oder eine Gruppe ist, die ausgewählt ist aus: 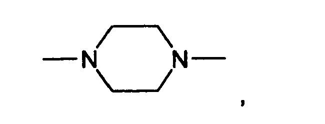 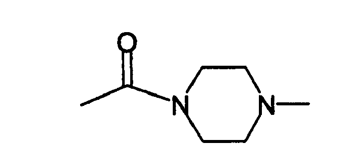 und 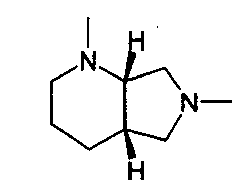
U und B unabhängig ein zweiwertiger Rest sind, der aus -N(R¹⁵)-, -O-, -S(O)_{z}-, -N(R¹⁵)C(O)-, -C(O)N(R¹⁵)- und -N[C(O)R¹⁵]- ausgewählt ist;
W -C(R¹⁶)- oder ein Stickstoffatom ist;
d 0 oder eine ganze Zahl von 1 bis 5 ist;
e eine ganze Zahl von 2 bis 4 ist;
j und z jeweils unabhängig ganze Zahlen von 0 bis 2 sind;
w eine ganze Zahl von 0 bis 4 ist;
t 2 oder 3 ist;
v eine ganze Zahl von 1 bis 8 ist;
oder ein pharmazeutisch akzeptables Derivat davon.

2. Verbindung gemäß Anspruch 1, worin A -N(R⁷)-CH₂- ist.

3. Verbindung gemäß Anspruch 1 oder 2, worin X -O(CH₂)₂NH- oder -O(CH₂)₂O- ist.

4. Verbindung gemäß einem der vorhergehenden Ansprüche, worin d 2 ist.

5. Verbindung gemäß einem der vorhergehenden Ansprüche, worin R⁸ eine heterocyclische Gruppe der folgenden Formel ist: 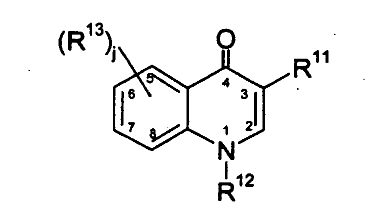 worin der Heterocyclus in der 6- oder 7-Stellung gebunden ist und j, R¹¹, R¹² und R¹³ wie in Anspruch 1 definiert sind.

6. Verbindung gemäß Anspruch 1 wie in einem der Beispiele 1 bis 8 definiert oder ein pharmazeutisch akzeptables Derivat davon.

7. Verbindung, die aus:
4"-(S)-{3-[2-(3-Carboxy-7-chlor-1-cyclopropyl-4-oxo-1,4-dihydro-chinolin-6-ylamino)-ethoxy]-propionylamino}-4"-desoxyazithromycin;
4"-(R)-{3-[2-(3-Carboxy-7-chlor-1-cyclopropyl-4-oxo-1,4-dihydro-chinolin-6-ylamino)-ethoxy]-propionylamino}-4"-desoxyazithromycin;
4"-(S)-{3-[2-(3-Carboxy-7-chlor-1-cyclopropyl-4-oxo-1,4-dihydro-chinolin-6-yloxy)-ethoxy]-propionylamino}-4"-desoxyazithromycin;
4"-(S)-{3-[2-(3-Carboxy-1-cyclopropyl-4-oxo-1,4-dihydro-chinolin-6-yloxy)-ethoxy]-propionylamino}-4"-desoxyazithromycin 11,12-cyclisches Carbonat und
4"-(3-{[2-(7-Chlor-1-cyclopropyl-3-methoxycarbonyl-4-oxo-1,4-dihydrochinolin-6-ylamino)-ethyl]-methylamino}-propionylamino)-4"-desoxyazithromycin ausgewählt ist;
oder ein pharmazeutisch akzeptables Derivat davon.

8. Verbindung, die aus:
4"-(S)-{3-[2-(3-Carboxy-7-chlor-1-cyclopropyl-4-oxo-1,4-dihydro-chinolin-6-ylamino)-ethoxy]-propionylamino}-4"-desoxyazithromycin,
4"-(R)-{3-[2-(3-Carboxy-7-chlor-1-cyclopropyl-4-oxo-1,4-dihydro-chinolin-6-ylamino)-ethoxy]-propionylamino}-4"-desoxyazithromycin,
4"-(S)-{3-[2-(3-Carboxy-7-chlor-1-cyclopropyl-4-oxo-1,4-dihydro-chinolin-6-yloxy)-ethoxy]-propionylamino}-4"-desoxyazithromycin,
4"-(R)-{3-[2-(3-Carboxy-7-chlor-1-cyclopropyl-4-oxo-1,4-dihydro-chinolin-6-yloxy)-ethoxy]-propionylamino}-4"-desoxyazithromycin,
4"-(R)-{3-[2-(3-Carboxy-1-cyclopropyl-4-oxo-1,4-dihydro-chinolin-6-yloxy)-ethoxy]-propionylamino}-4"-desoxyazithromycin,
4"-(R)-{3-[2-(3-Carboxy-1-cyclopropyl-4-oxo-1,4-dihydro-chinolin-6-yloxy)-ethoxy]-propionylamino}-4"-desoxyazithromycin 11,12-cyclisches Carbonat
4"-(S)-{3-[2-(3-Carboxy-1-cyclopropyl-4-oxo-1,4-dihydro-chinolin-6-yloxy)-ethoxy]-propionylamino}-4"-desoxyazithromycin 11,12-cyclisches Carbonat und
4"-(3-{[2-(7-Chlor-1-cyclopropyl-3-methoxycarbonyl-4-oxo-1,4-dihydro-chinolin-6-ylamino)-ethyl]-methylamino}-propionylamino)-4"-desoxyazithromycin ausgewählt ist,
oder ein pharmazeutisch akzeptables Derivat davon.

9. Verfahren zur Herstellung einer Verbindung gemäß Anspruch 1, das die folgenden Schritte umfaßt:
a) Umsetzen einer Verbindung der Formel (II) 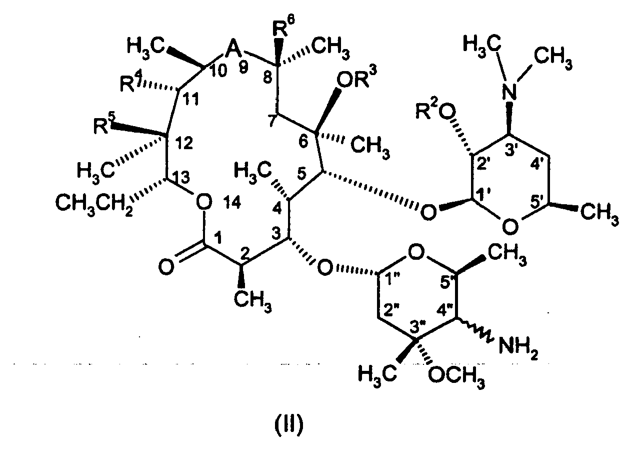
HOC(O)(CH₂)_{d}X^{a}R^{8a} (III)
mit einem geeigneten aktivierten Derivat der Säure (III), worin X^{a} und R^{8a} X und R⁸ wie in Anspruch definiert oder zu X und R⁸ umwandelbare Gruppen sind, zur Herstellung einer Verbindung der Formel (I), worin d eine ganze Zahl von 1 bis 5 ist;
b) Umsetzen einer Verbindung der Formel (II) mit einem geeigneten aktivierten Derivat der Carbonsäure HOC(O)N(R¹⁵)(CH₂)ᵥB^{a}R^{8a} (IV) zur Herstellung einer Verbindung der Formel (I), worin d 0 ist und U -C(O)N(R¹⁵)- ist;
c) Umsetzen einer Verbindung der Formel (II) mit einem Isocyanat OCN(CH₂)ᵥB^{a}R^{8a} zur Herstellung einer Verbindung der Formel (I), worin d 0 ist und U -NHist;
d) Umsetzen einer Verbindung der Formel (II) mit einem Carbamoylchlorid ClC(O)N(R¹⁵)(CH₂)ᵥB^{a}R^{8a} zur Herstellung einer Verbindung der Formel (I), worin d 0 ist und U -N(R¹⁵)- ist;
e) Umsetzen einer Verbindung der Formel (II) mit einem Chlorformiat ClOC(O)O(CH₂)ᵥB^{a}R^{8a} zur Herstellung einer Verbindung der Formel (I), worin d 0 ist und U -O- ist;
f) Umsetzen einer Verbindung der Formel (V): 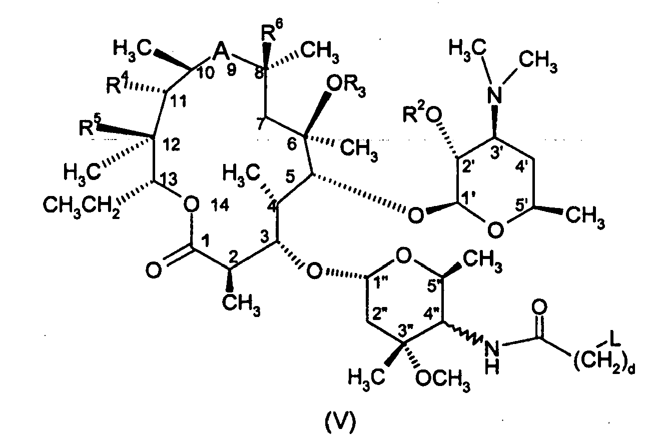 mit einer Verbindung der Formel X^{a}R^{8a} (VI), worin R^{8a} R⁸ wie in Anspruch 1 definiert oder eine zu R⁸ umwandelbare Gruppe ist und X^{a} -U(CH₂)ᵥ- oder -U(CH₂)ᵥB- oder eine zu -U(CH₂)ᵥ- oder -U(CH₂)ᵥB-umwandelbare Gruppe ist, worin U eine aus -N(R¹⁵)- und -S- ausgewählte Gruppe ist und L eine geeignete Abgangsgruppe ist, zur Herstellung einer Verbindung der Formel (I), worin U eine aus -N(R¹⁵)- und -S-ausgewählte Gruppe ist;
g) Umwandeln einer Verbindung der Formel (I) zu einer anderen Verbindung der Formel (I),
und danach, nach Bedarf, Unterwerfen der resultierenden Verbindung einem oder mehreren der folgenden Arbeitsgänge:
i) Entfernung der Schutzgruppe R²,
ii) Umwandlung von X^{a}R^{8a} zur XR⁸,
iii) Umwandlung von B^{a}R^{8a} zur BR⁸ und
iv) Umwandlung der resultierenden Verbindung der Formel (I) zu einem pharmazeutisch akzeptablen Derivat davon.

10. Verbindung gemäß einem der Ansprüche 1 bis 8 zur Verwendung in der Therapie.

11. Verwendung einer Verbindung gemäß einem der Ansprüche 1 bis 8 in der Herstellung eines Medikaments zur Verwendung in der Behandlung oder Prophylaxe von systemischen oder topischen mikrobiellen Infektionen in einem menschlichen oder tierischen Körper.

12. Pharmazeutische Zusammensetzung, die wenigstens eine Verbindung gemäß einem der Ansprüche 1 bis 8 in Verbindung mit einem pharmazeutisch akzeptablen Exzipienten, Verdünnungsmittel und/oder Träger umfaßt.

13. Verbindung der Formel (IA) 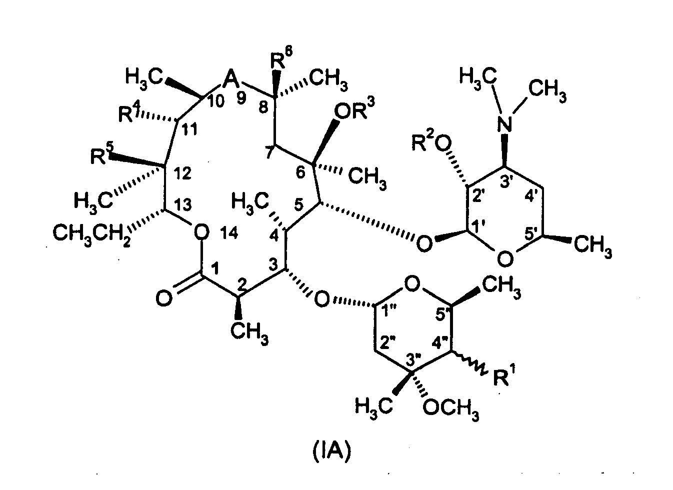 worin
A ein zweiwertiger Rest ist, der aus -C(O)NH-, -NHC(O)-, -N(R⁷)-CH₂- und -CH₂-N(R⁷)- ausgewählt ist;
R¹ -NHC(O)(CH₂)_{d}XR⁸ ist;
R² Wasserstoff ist;
R³ Wasserstoff, C₁₋₄-Alkyl oder gegebenenfalls mit 9-bis 10-gliedrigem kondensiertem bicyclischem Heteroaryl substituiertes C₃₋₆-Alkenyl ist;
R⁴ Hydroxy, gegebenenfalls mit 9- bis 10-gliedrigem kondensiertem bicyclischem Heteroaryl substituiertes C₃₋₆-Alkenyloxy oder gegebenenfalls mit C₁₋₆-Alkoxy oder -O(CH₂)ₑNR⁷R⁹ substituiertes C₁₋₆-Alkoxy ist;
R⁵ Hydroxy ist oder
R⁴ und R⁵ zusammen mit den dazwischenliegenden Atomen eine cyclische Gruppe mit der folgenden Struktur bilden: 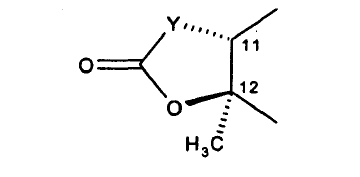 worin Y ein zweiwertiger Rest ist, der aus -CH₂-, -CH(CN)-, -O-, -N(R¹⁰)- und -CH(SR¹⁰)- ausgewählt ist, mit der Maßgabe, daß dann, wenn A -NHC(O)-, -N(R⁷)-CH₂- oder -CH₂-N(R⁷)- ist, Y -O- ist;
R⁶ Wasserstoff oder Fluor ist;
R⁷ Wasserstoff oder C₁₋₆-Alkyl ist;
R⁸ eine heterocyclische Gruppe mit der folgenden Struktur ist: 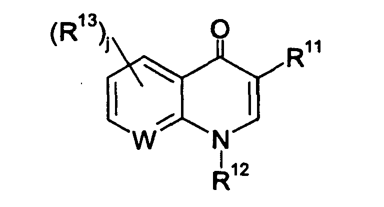 oder 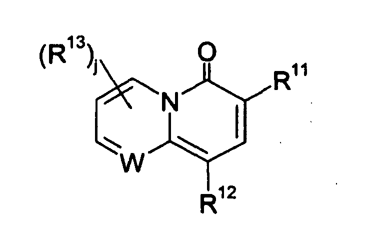
R⁹ Wasserstoff oder C₁₋₆-Alkyl ist;
R¹⁰ Wasserstoff oder C₁₋₄-Alkyl ist, das mit einer Gruppe substituiert ist, die aus gegebenenfalls substituiertem Phenyl, gegebenenfalls substituiertem 5- oder 6-gliedrigem Heteroaryl und gegebenenfalls substituiertem 9- bis 10-gliedrigem kondensiertem bicyclischem Heteroaryl ausgewählt ist;
R¹¹ Wasserstoff, -C(O)OR¹⁴, -C(O)NHR¹⁴ oder -C(O)CH₂NO₂ ist;
R¹² Wasserstoff, C₁₋₄-Alkyl, das gegebenenfalls mit Hydroxy oder C₁₋₄-Alkoxy substituiert ist, C₃₋₇-Cycloalkyl oder gegebenenfalls substituiertes Phenyl oder Benzyl ist;
R¹³ Halogen, C₁₋₄-Alkyl, C₁₋₄-Thioalkyl, C₁₋₄-Alkoxy, -NH₂, -NH(C₁₋₄-Alkyl) oder -N(C₁₋₄-Alkyl)₂ ist;
R¹⁴ Wasserstoff oder C₁₋₆-Alkyl ist, das gegebenenfalls mit bis drei Gruppen substituiert ist, die unabhängig aus Halogen, C₁₋₄-Alkoxy, -OC(O)C₁₋₆-Alkyl und -OC(O) OC₁₋₆-Alkyl ausgewählt sind;
R¹⁵ Wasserstoff, C₁₋₄-Alkyl, C₃₋₇-Cycloalkyl, gegebenenfalls substituiertes Phenyl oder Benzyl, Acetyl oder Benzoyl ist;
R¹⁶ Wasserstoff oder R¹³ ist oder R¹⁶ und R¹² unter Bildung des zweiwertigen Restes -O(CH₂)₂- oder -(CH₂)ₜ- verbunden sind;
X -U(CH₂)ᵥB-, -U(CH₂)ᵥ- oder eine Gruppe ist, die ausgewählt ist aus: 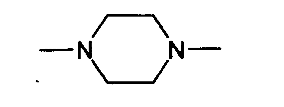 und 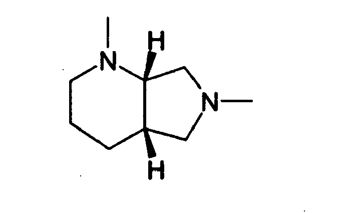
U und B unabhängig ein zweiwertiger Rest sind, der aus -N(R¹⁵)-, -O-, -S(O)_{z}-, -N(R¹⁵)C(O)-, -C(O)N(R¹⁵)- und -N[C(O)R¹⁵]- ausgewählt ist;
W -C(R¹⁶)- oder ein Stickstoffatom ist;
d 0 oder eine ganze Zahl von 1 bis 5 ist;
e eine ganze Zahl von 2 bis 4 ist;
j und z jeweils unabhängig ganze Zahlen von 0 bis 2 sind;
t 2 oder 3 ist;
v eine ganze Zahl von 1 bis 8 ist;
oder ein pharmazeutisch akzeptables Derivat davon.

## Revendications

1. Composé de formule (I) : 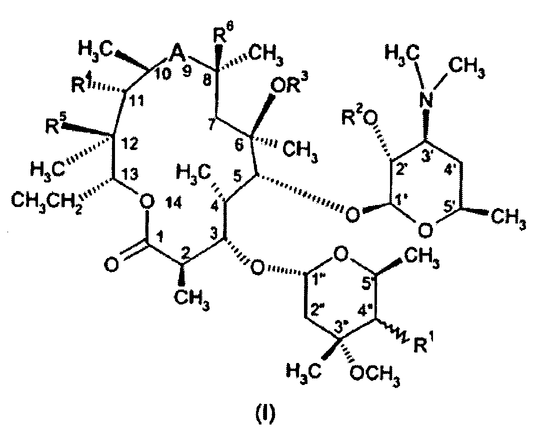 dans laquelle
A est un radical bivalent choisi parmi -C(O)NH-, - NHC (O) -, -N (R⁷) -CH₂- et -CH₂N (R⁷) - ;
R¹ est -NHC (O) (CH₂)_{d}XR⁸;
R² est un atome d'hydrogène ;
R³ est un atome d'hydrogène, un groupe alkyle en C₁₋₄, ou alcényle en C₃₋₆ facultativement substitué par un groupe hétéroaryle bicyclique fusionné à 9 à 10 chaînons ;
R⁴ est un groupe hydroxy, alcényloxy en C₃₋₆ facultativement substitué par un groupe hétéroaryle bicyclique fusionné à 9 à 10 chaînons, ou un groupe alcoxy en C₁₋₆ facultativement substitué par un groupe alcoxy en C₁₋₆ ou -O (CH₂) ₑNR⁷R⁹,
R⁵ est un groupe hydroxy, ou
R⁴ et R⁵ pris conjointement avec les atomes intervenants forment un groupe cyclique ayant la structure suivante : 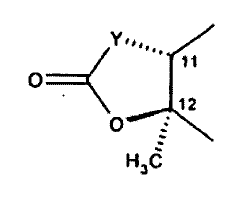 dans laquelle Y est un radical bivalent choisi parmi -CH₂-, -CH(CN)-, -O-, -N(R¹⁰)- et -CH(SR¹⁰)-, à condition que lorsque A est -NHC(O)-, -N (R⁷) -CH₂- ou - CH₂-N(R⁷)-, Y soit -O- ;
_{R}⁶ est un atome d'hydrogène ou de fluor ;
R⁷ est un atome d'hydrogène ou un groupe alkyle en C₁₋₆ ;
R⁸ est un groupe hétérocyclique ayant la structure suivante : 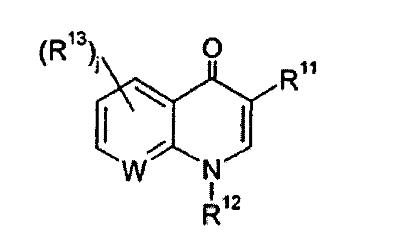 ou 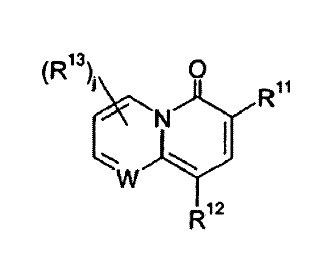
R⁹ est un atome d'hydrogène ou un groupe alkyle en C₁₋₆;
R¹⁰ est un atome d'hydrogène ou un groupe alkyle en C₁₋₄ facultativement substitué par un groupe choisi parmi un groupe phényle facultativement substitué, un groupe hétéroaryle à 5 ou 6 chaînons facultativement substitué et un groupe hétéroaryle bicyclique fusionné à 9 à 10 chaînons facultativement substitué ;
R¹¹ est un atome d'hydrogène, -C(O)OR¹⁴, -C(O)NHR¹⁴, -C(O)CH₂NO₂ ou -C(O)CH₂SO₂R⁷;
R¹² est un atome d'hydrogène, un groupe alkyle en C₁₋₄ facultativement substitué par un groupe hydroxy ou un groupe alcoxy en C₁₋₄, un groupe cycloalkyle en C₃₋₇, ou un groupe phényle ou benzyle facultativement substitué ;
R¹³ est un atome d'halogène, un groupe alkyle en C₁₋₄, thioalkyle en C₁₋₄, alcoxy en C₁₋₄, -NH₂, -NH (alkyle en C₁₋₄) ou N (alkyle en C₁₋₄)₂ ;
R¹⁴ est un atome d'hydrogène,
un groupe alkyle en C₁₋₆ facultativement substitué par jusqu'à 3 groupes indépendamment choisis parmi un atome d'halogène, un groupe cyano, alcoxy en C₁₋₄ facultativement substitué par un groupe phényle ou un groupe alcoxy en C₁₋₄, un groupe -C(O)(alkyle en C₁₋₆), - C(O)O(alkyle en C₁₋₆), -OC(O) (alkyle en C₁₋₆), - OC(O)O(alkyle en C₁₋₆), -C(O)NR¹⁷R¹⁸, NR¹⁷R¹⁸ et phényle facultativement substitués par un groupe nitro ou - C(O)O(alkyle en C₁₋₆),
-(CH₂)_{w}(cycloalkyle en C₃₋₇),
- (CH₂)_{w}hétérocyclyle,
-(CH₂)_{w}hétéroaryle,
-(CH₂)_{w}aryle,
alcényle en C₃₋₆, ou
alcynyle en C₃₋₆ ;
R¹⁵ est un atome d'hydrogène, un groupe alkyle en C₁₋₄, cycloalkyle en C₃₋₇, un groupe phényle ou benzyle facultativement substitué, un groupe acétyle ou benzoyle ;
R ¹⁶ est un atome d'hydrogène ou R¹³, ou R¹⁶ et R¹² sont liés pour former le radical bivalent -O(CH₂)₂ ou - (CH₂)ₜ- ;
R¹⁷ et R¹⁸ sont chacun indépendamment un atome d'hydrogène ou un groupe alkyle en C₁₋₆ facultativement substitué par un groupe phényle ou -C(O)O(alkyle en C₁-6), ou
R¹⁷ et R¹⁸, conjointement avec l'atome d'azote auquel ils sont liés, forment un groupe hétérocyclique à 5 ou 6 chaînons contenant facultativement un hétéroatome supplémentaire choisi parmi l'oxygène, l'azote et le soufre ;
X est -U(CH₂)ᵥB-, -U(CH₂)ᵥ- ou un groupe choisi parmi : 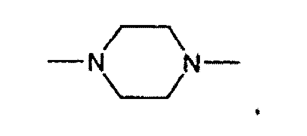 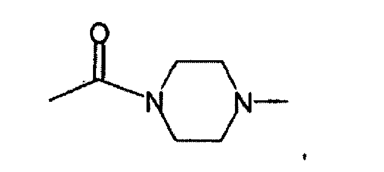 et 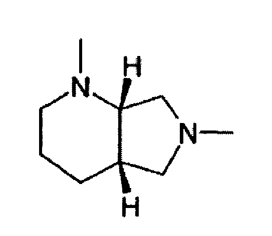
U et B sont indépendamment un radical bivalent choisi parmi -N(R¹⁵)-, -O-, -S(O)_{z}-, -N(R¹⁵)C(O)-, - C(O)N(R¹⁵)- et -N[C(O)R¹⁵]- ;
W est -C(R¹⁶)- ou un atome d'azote ;
d est 0 ou un entier de 1 à 5 ;
e est un entier de 2 à 4 ;
j et z sont chacun indépendamment des entiers de 0 à 2 ;
w est un entier de 0 à 4 ;
t est 2 ou 3 ;
v est un entier de 1 à 8 ;
ou un dérivé pharmaceutiquement acceptable de celui-ci.

2. Composé selon la revendication 1, dans lequel A est -N(R⁷) -CH₂-.

3. Composé selon la revendication 1 ou la revendication 2, dans lequel X est -O(CH₂)₂NH- ou - O(CH₂)₂O-.

4. Composé selon l'une quelconque des revendications précédentes, dans lequel d est 2.

5. Composé selon l'une quelconque des revendications précédentes, dans lequel R⁸ est un groupe hétérocyclique ayant la formule suivante : 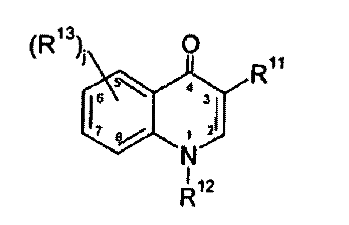 dans laquelle le groupe hétérocyclique est lié à la position 6 ou 7 et j, R¹¹, R¹² et R¹³ sont tels que définis dans la revendication 1.

6. Composé selon la revendication 1 tel que défini dans l'un quelconque des exemples 1 à 8, ou un dérivé pharmaceutiquement acceptable de celui-ci.

7. Composé choisi parmi :
la 4"-(S)-{3-[2-(3-carboxy-7-chloro-1-cyclopropyl-4-oxo-1,4-dihydro-quinoléin-6-ylamino)éthoxy]-propionylamino}-4"-désoxyazithromycine ;
la 4"-(R)-{3-[2-(3-carboxy-7-chloro-1-cyclopropyl-4-oxo-1,4-dihydro-quinoléin-6-ylamino)éthoxy]-propionylamino}-4"-désoxyazithromycine ;
la 4"-(S)-{3-[2-(3-carboxy-7-chloro-1-cyclopropyl-4-oxo-1,4-dihydro-quinoléin-6-yloxy)-éthoxy]propionylamino}-4"-désoxyazithromycine ;
le carbonate 11,12-cyclique de 4"-(S)-{3-[2-(3-carboxy-1-cyclopropyl-4-oxo-1,4-dihydro-quinoléin-6-yloxy)éthoxy)propionylamino}-4"-désoxyazithromycine ; et
la 4"-(3-{[2-(7-chloro-1-cyclopropyl-3-méthoxycarbonyl-4-oxo-1,4-dihydro-quinoléin-6-ylamino)-éthyl)-méthyl-amino}-propionylamino)-4"-désoxyazithromycine ;
ou un dérivé pharmaceutiquement acceptable de celui-ci.

8. Composé choisi parmi :
la 4"-(S)-{3- [2-(3-carboxy-7-chloro-1-cyclopropyl-4-oxo-1,4-dihydro-quinoléin-6-ylamino)-éthoxy -propionylamino}-4"-désoxyazithromycine
la 4"-(R)-{3- [2-(3-carboxy-7-chloro-1-cyclopropyl-4-oxo-1,4-dihydro-quinoléin-6-ylamino)-éthoxy -propionylamino}-4"-désoxyazithromycine
la 4"-(S)-{3- [2-(3-carboxy-7-chloro-1-cyclopropyl-4-oxo-1,4-dihydro-quinoléin-6-yloxy)-éthoxy -propionylamino}-4"-désoxyazithromycine
la 4"-(R)-{3- [2-(3-carboxy-1-cyclopropyl-4-oxo-1,4-dihydro-quinoléin-6-yloxy)-éthoxy -propionylamino}-4"-désoxyazithromycine
le carbonate 11, 12-cyclique de 4"-(R)-{3- [2-(3-carboxy-1-cyclopropyl-4-oxo-1,4-dihydro-quinoléin-6-yloxy)-éthoxy -propionylamino}-4"-désoxyazithromycine
le carbonate 11, 12-cyclique de 4"-(S)-{3- [2-(3-carboxy-1-cyclopropyl-4-oxo-1,4-dihydro-quinoléin-6-yloxy)-éthoxy -propionylamino}-4"-désoxyazithromycine
la 4"-(3- {[2-(7-chloro-1-cyclopropyl-3-méthoxycarbonyl-4-oxo-1,4-dihydro-quinoléin-6-ylamino)-éthyl]-méthyl-amino}-propionylamino}-4"-désoxyazithromycine
ou un dérivé pharmaceutiquement acceptable de celui-ci.

9. Processus de préparation d'un composé selon la revendication 1, comprenant :
a) la réaction d'un composé de formule (II) 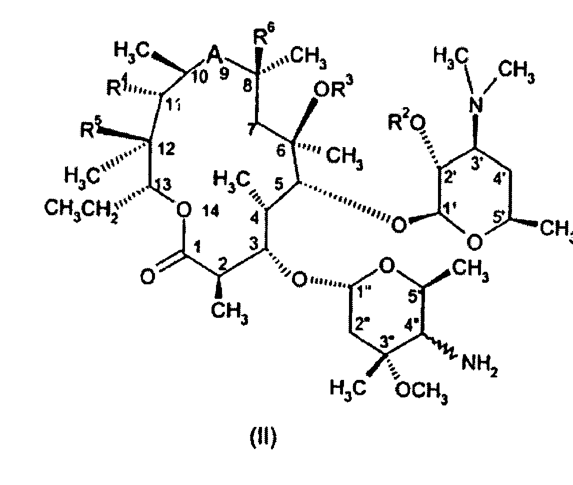
HOC(O)(CH₂)_{d}X^{a}R^{8a} (III)
avec un dérivé activé adapté de l'acide (III), dans laquelle X^{a} et R^{8a} sont X et R⁸ tels que définis dans la revendication 1 ou des groupes pouvant être convertis en X et R⁸ pour produire un composé de formule (I) dans laquelle d est un entier de 1 à 5 ;
b) la réaction d'un composé de formule (II) avec un dérivé activé adapté de l'acide carboxylique HOC(O)N(R¹⁵) (CH₂)ᵥB^{a}R^{8a} (IV) pour produire un composé de formule (I) dans laquelle d est 0 et U est - C(O)N(R¹⁵)-;
c) la réaction d'un composé de formule (II) avec un isocyanate OCN(CH₂)ᵥB^{a}R^{8a} pour produire un composé de formule (I) dans laquelle d est 0 et U est -NH- ;
d) la réaction d'un composé de formule (II) avec un chlorure de carbamoyle ClC(O)N(R¹⁵)(CH₂)ᵥB^{a}R^{8a} pour produire un composé de formule (I) dans laquelle d est 0 et U est -N(R¹⁵)-;
e) la réaction d'un composé de formule (II) avec un chloroformate ClOC(O)O(CH₂)ᵥB^{a}R^{8a} pour produire un composé de formule (I) dans laquelle d est 0 et U est -0- ;
f) la réaction d'un composé de formule (V) 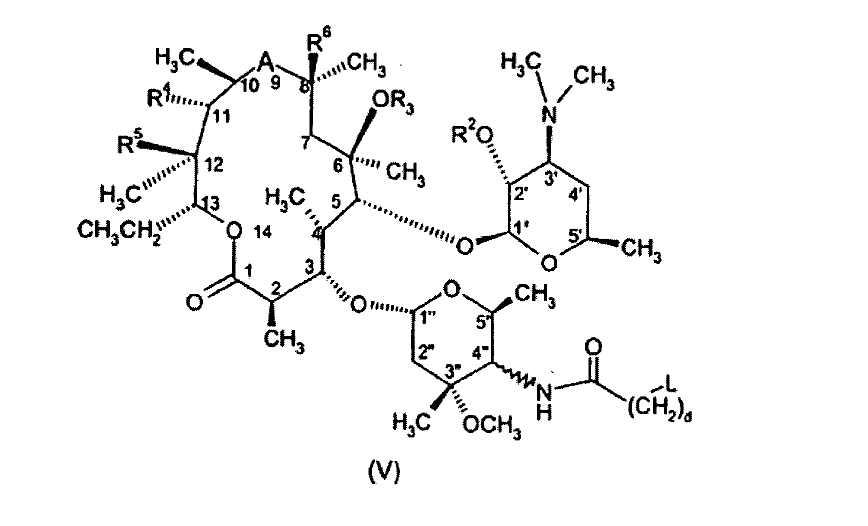 avec un composé de formule X^{a}R^{8a} (VI) , dans laquelle R^{8a} est R⁸ tel que défini dans la revendication 1, ou un groupe pouvant être converti en R⁸ et X^{a} est - U(CH₂)ᵥ- ou -U (CH₂)ᵥB-, ou un groupe pouvant être converti en -U(CH₂)ᵥ- ou -U(CH₂)ᵥB-, dans lequel U est un groupe choisi parmi -N(R¹⁵)- et -S-, et L est un groupe partant adapté, pour produire un composé de formule (I) dans laquelle U est un groupe choisi parmi -N(R¹⁵) - et -S- ; ou
g) la conversion d'un composé de formule (I) en un autre composé de formule (I),
et après ceci, au besoin, la soumission du composé résultant à une ou plusieurs des opérations suivantes :
i) l'élimination du groupe de protection R²,
ii) la conversion de X^{a}R^{8a} en XR⁸,
iii) la conversion de B^{a}R^{8a} en BR⁸, et
iv) la conversion du composé résultant de formule (I) en un dérivé pharmaceutiquement acceptable de celui-ci.

10. Composé selon l'une quelconque des revendications 1 à 8 destiné à être utilisé en thérapie.

11. Utilisation d'un composé selon l'une quelconque des revendications 1 à 8, dans la fabrication d'un médicament destiné à être utilisé dans le traitement ou la prophylaxie d'infections microbiennes systémiques ou topiques dans un corps humain ou animal.

12. Composition pharmaceutique comprenant au moins un composé selon l'une quelconque des revendications 1 à 8, en association avec un excipient, un diluant et/ou un vecteur pharmaceutiquement acceptable.

13. Composé de formule (IA) : 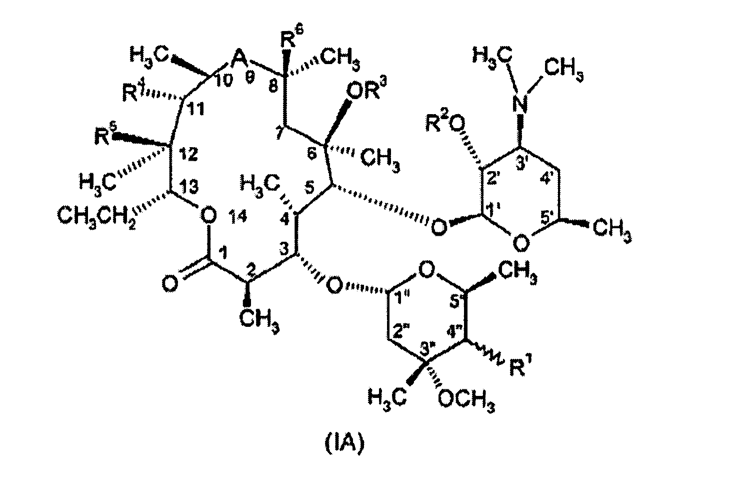 dans laquelle
A est un radical bivalent choisi parmi -C(O)NH-, - NHC(O)-, -N(R⁷)-CH₂- et -CH₂N(R⁷)- ;
R¹ est -NHC(O)(CH₂)_{d}XR⁸ ;
R² est un atome d'hydrogène ;
R³ est un atome d'hydrogène, un groupe alkyle en C₁₋₄, ou alcényle en C₃₋₆ facultativement substitué par un groupe hétéroaryle bicyclique fusionné à 9 à 10 chaînons ;
R⁴ est un groupe hydroxy, alcényloxy en C₃₋₆ facultativement substitué par un groupe hétéroaryle bicyclique fusionné à 9 à 10 chaînons, ou un groupe alcoxy en C₁₋₆ facultativement substitué par un groupe alcoxy en C₁₋₆ ou -O(CH₂)ₑNR⁷R⁹,
R⁵ est un groupe hydroxy, ou
R⁴ et R⁵ pris conjointement avec les atomes intervenants forment un groupe cyclique ayant la structure suivante : 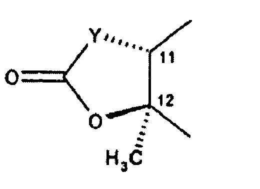 dans laquelle Y est un radical bivalent choisi parmi -CH₂-, -CH(CN)-, -O-, -N(R¹⁰)- et -CH(SR¹⁰)-, à condition que lorsque A est -NHC(O)-, -N(R⁷)-CH₂- ou - CH₂-N(R⁷)-, Y soit -O- ;
R⁶ est un atome d'hydrogène ou de fluor ;
R⁷ est un atome d'hydrogène ou un groupe alkyle en C₁₋₆;
R⁸ est un groupe hétérocyclique ayant la structure suivante: 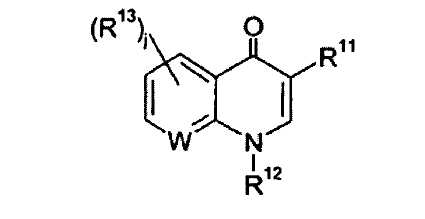 ou 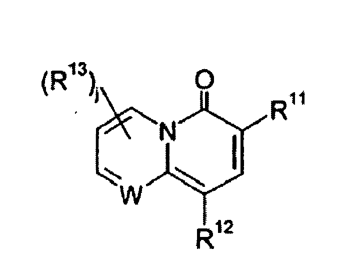
R⁹ est un atome d'hydrogène ou un groupe alkyle en C₁₋₆ ;
R¹⁰ est un atome d'hydrogène ou un groupe alkyle en C₁₋₄ substitué par un groupe choisi parmi un groupe phényle facultativement substitué, un groupe hétéroaryle à 5 ou 6 chaînons facultativement substitué et un groupe hétéroaryle bicyclique fusionné à 9 à 10 chaînons facultativement substitué ;
R¹¹ est un atome d'hydrogène, -C(O)OR¹⁴, -C (O) NHR¹⁴ ou -C(O)CH₂NO₂ ;
R¹² est un atome d'hydrogène, un groupe alkyle en C₁₋₄ facultativement substitué par un groupe hydroxy ou un groupe alcoxy en C₁₋₄, cycloalkyle en C₃₋₇, ou un groupe phényle ou benzyle facultativement substitué ;
R¹³ est un atome d'halogène, un groupe alkyle en C₁₋₄, thioalkyle en C₁₋₄, alcoxy en C₁₋₄, -NH₂, -NH (alkyle en C₁₋₄) ou -N (alkyle en C₁₋₄)₂ ;
R¹⁴ est un atome d'hydrogène, un groupe alkyle en C₁₋₆ facultativement substitué par jusqu'à 3 groupes indépendamment choisis parmi un atome d'halogène, un groupe alcoxy en C₁₋₄, -OC(O) (alkyle en C₁₋₆) et - OC(O)O(alkyle en C₁₋₆) ;
R¹⁵ est un atome d'hydrogène, un groupe alkyle en C₁₋₄, cycloalkyle en C₃₋₇, un groupe phényle ou benzyle facultativement substitué, un groupe acétyle ou benzoyle ;
R¹⁶ est un atome d'hydrogène ou R¹³, ou R¹⁶ et R¹² sont liés pour former le radical bivalent -O(CH₂)₂ ou - (CH₂) ₜ- ;
X est -U(CH₂)ᵥB-, -U(CH₂)ᵥ- ou un groupe choisi parmi : 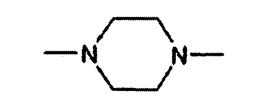 et 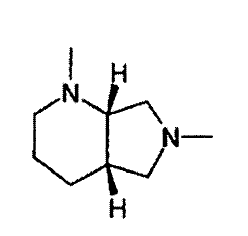
U et B sont indépendamment un radical bivalent choisi parmi -N(R¹⁵)-, -O-, -S(O)_{z}-, -N(R¹⁵)C(O)-, - C(O)N(R¹⁵)- et -N[C(O)R¹⁵]- ;
W est -C(R¹⁶)- ou un atome d'azote ;
d est 0 ou un entier de 1 à 5 ;
e est un entier de 2 à 4 ;
j et z sont chacun indépendamment des entiers de 0 à 2 ;
t est 2 ou 3 ;
v est un entier de 2 à 8 ;
ou un dérivé pharmaceutiquement acceptable de celui-ci.
